# EUROPEAN PATENT SPECIFICATION

(11) **EP 1 373 529 B1**
(45) Date of publication and mention of the grant of the patent: **23.04.2008**
(21) Application number: 02702181.5
(22) Date of filing: 25.02.2002
(51) Int. Cl.: C12N 15/81, C12N 15/12, C12N 1/21, C12Q 1/02

(54) **TAU-OPATHY MODEL**
TAU-OPATHY MODELL
MODELE DE TAU-OPATHIE

(30) Priority: 26.02.2001 GB 0104685
(43) Date of publication of application: 02.01.2004
(73) Proprietor: NV reMynd, 3000 Leuven (BE)
(72) Inventor: VAN LEUVEN, Freddy, B-3210 Linden (BE); WINDERICKX, Joris, 3012 Wilsele (BE)
(74) Representative: Bird, Ariane
(86) International application number: PCT/BE2002/000022
(87) International publication number: WO 2002/068663

(56) References cited:
- WO-A-01/02552
- WO-A-99/29891
- US-A- 5 952 217
- US-A- 5 958 721
- US-A- 5 994 084
- SONG SCHAOCHUEN ET AL: "Proteolytic processing and degradation of human presenilin-1 expressed in yeast." NEUROSCIENCE LETTERS, vol. 282, no. 1-2, 17 March 2000 (2000-03-17), pages 65-68, XP002201655 ISSN: 0304-3940
- ZHOU X Z (REPRINT) ET AL: "Pin1-dependent prolyl isomerization regulates dephosphorylation of Cdc25C and tau proteins" MOLECULAR CELL, (OCT 2000) VOL. 6, NO. 4, PP. 873-883. PUBLISHER: CELL PRESS, 1050 MASSACHUSETTES AVE, CIRCULATION DEPT, CAMBRIDGE, MA 02138. ISSN: 1097-2765., XP002201656
- ESCHER D (REPRINT) ET AL: "ESBATech AG - Taking yeast from the brewery to drug discovery" CHIMIA, (APR 2000) VOL. 54, NO. 4, PP. 171-173. PUBLISHER: NEW SWISS CHEMICAL SOC, C/O NOVARTIS AG, K-25 1 45, CH-4002 BASEL, SWITZERLAND. ISSN: 0009-4293., XP001080002
- GEYSKENS I. ET AL.: "Expression of mammalian PKB partially complements deletion of the yeast protein kinase Sch9" NATO SCIENCE SERIES, vol. A316, 2000, pages 117-126, XP002201945 cited in the application

## Description

### FIELD OF THE INVENTION

The present invention relates to a yeast model for the tau-opathy as in Alzheimer's disease and other neurodegenerative disorders. This model of engineered yeast can be used in pharmaceutical screening and for modelling neurodegenerative diseases (e.g., Alzheimer's disease, frontotemporal dementia with Parkinsonism) and for in vivo modelling of protein tau biochemistry. It can be used as an assay, automated assay or high through put screening assay for identifying agents, compounds or chemical signals that directly or indirectly affect the biochemistry of tau (protein tau or tau-protein) and in particular of protein tau phosphorylation, comprising the steps of: growing the yeast cell line in appropriate media, said yeast cell comprising an introduced polynucleotide or DNA sequence, an allelic variant, minigene or a homologue thereof, that encodes for protein tau, protein tau isoforms or functional homologues thereof and expresses or overexpresses protein tau or functional homologues thereof and wherein said yeast cell comprising a protein kinase that is capable directly or indirectly of modulating of protein tau, adding the test compound or chemical signal to the media; and measuring the extent to which the protein tau or functional homologues thereof are phosphorylated.

Alzheimer's disease, a neurodegenerative disease which is targeted by screening assay of present invention, is the most common form of senile dementia, affecting approximately 5% of individuals over the age of 65 and 20% of those over the age of 80. It has been estimated that there are between 2.5 and 3 million patients suffering from Alzheimer's disease in the USA and up to 6 million in Europe. These figures will increase exponentially over the next decades as the proportion of elderly in the population increases exponentially and because the incidence of Alzheimer's disease itself increases exponentially with age.
To date, there are neither accurate methods for early diagnosis, nor any method or drug for the effective treatment of Alzheimer's disease. The development to market of a therapeutic intervention for this major human disease therefore represents a significant commercial opportunity.

### BACKGROUND OF THE INVENTION

Alzheimer's disease is a neurodegenerative disorder characterised histopathologically by the loss of synapses on particular groups of neurones and eventually in a later clinical stage, by the loss of these neurones themselves. Post-mortem pathology reveals the abundant presence of two principal lesions within the brain, named senile plaques and neurofibrillary tangles (Delacourte, 1999).

The mechanisms that cause the synaptic and neuronal loss are still not understood. Several different mutations in the gene coding for the amyloid precursor protein (APP) in some families with early onset familial Alzheimer's disease (EOFAD) supports a "amyloid first" hypothesis in which the extracellular deposition of amyloid peptides derived from APP is an early pathogenic event (for reviews see Hardy et al, 1998; Selkoe, 2000). In most other families with EOFAD , the disease is caused by mutations in the gene coding for Presenilin-1 (PS1), while in some rare families affected members carry a mutant Presenilin-2 gene (PS2). Increased amyloidogenic APP metabolism and deposition of amyloid peptides are regarded as a primary pathogenic event, however, which does not imply that a general consensus is reached on the primary pathogenic effect of and how such deposition of amyloid peptides in the brain would result in neurodegeneration and dementia.

Senile plaques are extracellular fibrillar deposits of amyloid peptides, surrounded by dystrophic neurites, which contain tau-aggregates. Amyloid deposits are found in the brain parenchym but also prominently in the vascular walls of the deeper cerebral blood vessels in all cases of Alzheimer's disease, be they early onset or late onset or sporadic cases. The amyloid deposits in senile plaques and in cerebral blood vessels contain amyloid peptides of 40 and of 42 amino acids in length. These are derived by proteolytic cleavage from the larger amyloid precursor protein (APP), a membrane-anchored glycoprotein, 100 to 110 kDa in size.

On the other hand, neurofibrillary tangles (NFT) are intra-neuronal inclusions of paired helical filaments (PHF) which themselves are aggregates of protein tau, a microtubule-associated protein. Protein tau refers to a set of up to 6 isoforms of this cytosolic phosphoprotein (about 60-70 kDa). Hyper-phosphorylation of tau is thought to represent the principal cause of its aggregation into PHF and all subsequent malfunctions in terms of cytoskeletal and synaptic stability, neurite outgrowth and axonal transport. Neurones containing neurofibrillary tangles are to be considered heavily compromised and not able to function normally. Neuronal death of such cells is evident from the presence of "ghost" tangles in brain of Alzheimer's disease patients, i.e. residues of dead tangle-bearing neurones from which essentially only the insoluble neurofibrillary tangles have been remained.

Alzheimer disease targets are currently studied by transgenic mice models for Alzheimer's disease (for reviews see Van Leuven 2000; Dewachter et al., 2000). These models focus on APP and PS1, because mutations in APP and PS1 cause EOFAD and because Down's patients (trisomy 21 on which the APP gene is located) develop classical Alzheimer's disease pathology in their 2^{nd} to 3^{rd} decade, due to the overexpression of APP by the gene-dosage effect. Single and double transgenic mice, i.e. APP and APP x PS1 transgenic mice in whom the transgenes contain one or more EOFAD clinical mutations, show a prominent and robust amyloid pathology phenotype. This recapitulates the amyloid pathology of AD patients almost exactly in terms of deposits of amyloid in the parenchym and in the cerebral blood vessels (Van Dorpe et al, 2000; Dewachter et al, 2000). Significantly, and very remarkably, although hyper-phosphorylation of tau is observed in the swollen neurites surrounding the neuritic plaques in these mice, no neurofibrillary tangles have been formed in the brain of these amyloid-bearing transgenic mice (Van Dorpe et al, 2000).

It is a matter of strong debate if the cognitive deficits demonstrated in the transgenic APP and APP × PS1 mice, are relevant for the cognitive deficits in the human Alzheimer patients. In brain of patients, the cognitive decline correlates much better with the presence and site of dystrophic neurites containing PHF and neurofibrillary tangles than with amyloid plaque deposition. A popular hypothesis holds that even though a primary pathogenic event must involve aberrant APP metabolism, the mechanism by which the neurone degenerates is essentially a dysfunction or disruption of the neuronal cytoskeleton. This impairs the axonal, and perhaps also dendritic transport, and is due to the hyper-phosphorylation of tau, which eventually also results in its deposition as PHF.

On the other hand, transgenic mice have been generated that overexpress human protein tau, either wild-type or a clinical mutant that give rise to another type of dementia, i.e. frontotemporal dementia with Parkinsonism linked to chromosome 17 (FTDP-17) (for review see Heutink, 2000).
Although these tau-transgenic mice do not develop an Alzheimer related phenotype, they demonstrate the massive and pathologic interference of human tau with axonal transport, causing "ballooning" of the axons, axonal degeneration and as a secondary phenomenon, muscular wasting and motoric problems(Spittaels et al, 1999, 2000). This phenotype is, most amazingly, "rescued" by GSK-3β in double transgenic mice, in which next to human tau also GSK-3β is expressed (Spittaels et al, 2000). In the double transgenic mice, the increased level of hyper-phosphorylation of protein tau correlates with decreased ballooning, alleviation of the axonopathy and of the motoric and muscular problems (Spittaels et al, 2000). This clearly indicates a duality in the role of GSK-3β and other kinases that are eventually (or obligatorily) implicated in the signalling pathways up- or down-stream of GSK-3β. These findings evidently complicate any existing scheme in which hyper-phosphorylation of tau is suggested or advocated to be the direct cause of the axonal problem. In this respect, the transgenic tau or GSK-3β mice are not suitable as models to elucidate the molecular mechanisms by which the tau-pathology is "triggered" and "executed". Such mechanisms must and can be studied in cellular paradigms, preferable as simple as possible in order to define the essential and fundamental features of signalling pathways and their phenotypic implications. Present invention discloses such cellular paradigm which besides ease of manipulation, both genetically and epi-genetically in terms of environmental and medium parameters also provides a simple system which moreover is an essential bonus for high-throughput screening purposes.

### SUMMARY OF THE INVENTION

This invention discloses engineered yeast cells that are models for aspects of Alzheimer's disease and for another neurodegenerative disease, such as frontotemporal dementia with Parkinsonism, in which said engineered yeast cells express a human wild-type or mutant protein tau isoform, by introducing a DNA sequence encoding one of these isoforms. A further aspect of the invention is that the engineered yeast cells express a human wild-type or mutant protein tau isoform, by introducing a DNA sequence encoding one of these isoforms and are also capable of expressing a tau-kinase that is a direct or indirect modulator of the phosphorylation state of the protein tau isoform expressed in the same cell. In a further aspect of the invention said engineered yeast cells contain introduced DNA sequences that encode and are capable of expressing the said protein tau and the said protein-tau kinase that are capable, directly or indirectly, to modulate the phosphorylation state of the protein tau. In yet another aspect of the invention said engineered yeast cells contain an introduced DNA sequence comprising a control sequence, correctly integrated to allow the expression of human protein tau, isoform protein tau or functional homologous thereof and also comprises a DNA sequence encoding and capable of expressing a protein tau kinase or other kinase, capable of direct or indirect modulation of the phosphorylation state of protein tau.

The DNA sequence of present invention encoding and capable of expressing a protein kinase that is capable, directly or indirectly, of modulating the phosphorylation-site of protein tau can be either endogenous, but can or may also be introduced to establish or bring about increased production of the chosen kinase, such as co-factored or accessory subunits or activators or modulators.

The invention includes also the progeny and all subsequent generations of the cells into which the said DNA sequence(s) were introduced.

The engineered yeast cells of present invention are used as a yeast model for a yeast model for the tau-opathy as in Alzheimer's disease and other neurodegenerative disorders. This model of engineered yeast can be used in pharmaceutical screening and for modelling neurodegenerative diseases (e.g., Alzheimer's disease, frontotemporal dementia with Parkinsonism) and for in vivo modelling of protein tau biochemistry. It can be used as an assay, automated assay or high through put screening assay for identifying agents, compounds or chemical signals that directly or indirectly affect the biochemistry of tau (protein tau or tau-protein) and in particular of protein tau phosphorylation, comprising the steps of: growing the yeast cell line in appropriate media, said yeast cell comprising an introduced polynucleotide or DNA sequence, an allelic variant, minigene or a homologue thereof, that encodes for protein tau, protein tau isoforms or functional homologues thereof and expresses or overexpresses protein tau or functional homologues thereof and wherein said yeast cell comprising a protein kinase that is capable directly or indirectly of modulating of protein tau, adding the test compound or chemical signal to the media; and measuring the extent to which the protein tau or functional homologues thereof are phosphorylated.

### ILLUSTRATIVE EMBODIMENTS OF THE INVENTION

The invention is based on the notion that modelling of all neuro-degenerative aspects of Alzheimer's disease is a most important and essential requirement.

### BRIEF DESCRIPTION OF THE FIGURES

Figure1:
   Heterologous protein tau expression in wild type S. cerevisiae. Western blot analysis of crude cell extracts from different wild type strains and isogenic mds1 deletion strains using the Tau-5 antibody to detect expression of either human tau-wt or tau-P301L (Panel A) and GFP-tau-wt or GFP-tau-P301 L fusion proteins (Panel B). Expression of GFP-fusions was further analyzed by fluorescence microscopy (Panel B)
Figure 2:
   Phosphorylation mapping. The phosphorylation map assay was performed on crude extracts derived from the wild type strain, the isogenic mds1 deletion strain and the isogenic pho85 deletion strain that express either human tau-wt or tau-P301L. Panel A: strains from the W303-1A genetic background. Panel B: strains from the BY4741 genetic background.
   Different antibodies were used as indicated; Tau-5 as phosphorylation-independent "pan"-tau antibody to visualize the total amount of tau-protein blotted, AT-8 and AD2 as antibodies that recognize different phosphorylated tau-epitopes, and Tau-1 as antibody that recognizes non-phosphorylated tau-epitopes.
Figure 3:
   Effect of PP2A overexpression on tau-phosphorylation. Western blot analysis of the W303-1A wild type strain or the same strain overexpressing the protein phosphatase Pph21. Immunodetection was performed using the phosphorylation independent antibody Tau-5 or the antibody that recognizes non-phosphorylated epitopes Tau-1 as indicated.
Figure 4:
   GSK3 complementation analysis. The human GSK3-beta was expressed in a yeast mds1 deletion strain to demonstrate functional complementation for tau-phosphorylation Phosphorylation of tau-wt and tau-P301 L was monitored in the W303-1A wild type, the mds1 deletion strain and the mds1 deletion strain expressing human GSK3-beta. Western blot analysis was performed on crude cell extracts using the AD2 antibody for which immunoreactivity is dependent on phosphorylation of the Ser-396 and Ser-404 epitopes . Immunodetection with Tau-5 served as control.
Figure 5:
   Validation of the yeast model to study signal transduction cascades with tau-phosphorylation as marker. Phosphorylation of heterologous expressed human protein tau-wt and tau-P301L was monitored in different strains deficient for kinases previously demonstrated to operate in inter-connecting signal transduction cascades in S.cerevisiae. The upper panels show phosphorylation mapping in the W303-1A sch9 deletion strain, the middle panels for the W303-1A yak1 deletion strain and the lower panels for strain deleted for the different Tpk subunits of PKA. Corresponding human homologues kinase activities are indicated between brackets. Immunodetection was performed using the antibodies AD2, Tau-5 and Tau-1.
Figure 6:
   Effect of heterologous expression of human protein tau on physiological processes in yeast. Panel A : The effect on tau expression on pseudohyphal differentation upon nitrogen limitation was monitored in the Σ1278 strain. As a control for full pseudohyphal differentiation, the strain transformed with the empty plasmid is included in the picture on the left. The inhibitory effect of tau-P301L expression or tau-wt expression are visualised respectively in the middle and the right picture. Panel B and C: The effect of expression of tau-wt and tau-P301 L on benomyl sensitivity of the BY4741 wild type strain (panel B), the isogenic mds1 deletion strain (panel B) and the isogenic pho85 deletions strain (B and C). Panel B shows the growth curves (OD6OO nm) in liquid cultures in the presence of 40 microgram benomyl as measured with the BIOSCREEN C workstation. Panel C shows the plate assay for growth of serial dilutions of the pho85 deletion strains transformed with, from left to right, the empty plasmid, tau-P301 L or tau-wt. The final benomyl concentrations in the plates are as indicated
Figure 7:
   Solubility assay of heterologous expressed human protein tau in S. cerevisiae. Sequentially obtained protein extracts obtained from the wild type BY4741 strain and the isogenic mds1 deletion strain expressing tau-wt or the pho85 deletion strain expressing either tau-wt or tau-P3041L ,as indicated, were analysed by western blot analysis using the Tau-5 antibody. The lanes are indicated and contain samples of the supernatant or the pellet obtained from subsequent extraction in high salt reassembly buffer (RAB), detergent containing radioimmunoprecipitation assay buffer (RIPA) or 70% formic acid (FA)

### TERMINOLOGY AND DEFINITIONS

For purposes of the present invention, the following terms are defined below.

The term "cell lines" is used in the present invention refers to eukaryotic cell lines preferably in the form of a cell line that is suitable for continuos culture, either in suspension or attached on a suitable carrier and is a recombinant cell that may be obtained by manipulation of cells using conventional techniques of recombinant DNA technology.

The term "yeast cell" herein is used to mention single-celled fungi of the phylum Ascomycota that reproduce by fission or budding and are capable of fermenting carbohydrates into alcohol and carbon dioxide. Yeast cells of the species Saccharomyces cerevisae are preferred for manipulation to incorporate DNA sequences in accordance with the present invention. Such cells do not normally express a protein tau but are capable of expressing human protein tau by introduction of a DNA sequence encoding human tau under the control of appropriate regulatory DNA sequences. The resulting DNA sequence is considered a "recombinant" DNA sequence or a "transgene".

The term "engineered yeast" is used herein to mention yeast cells, having a transgene or non-endogenous (i.e. heterologous) nucleic acid sequence present as a extrachromosomal element in stably integrated into its germ line DNA (i.e. in the genomic DNA). Heterologous nucleic acid is introduced into the germ line of such engineered by genetic manipulation

The term "introduced DNA sequence" is used herein to denote a DNA sequence that has been introduced into a cell and which may or may not be incorporated into the genome. The DNA sequence may be a sequence that is not endogenous to the chosen type of cell, that is endogenous but is not normally expressed by that cell or that is endogenous and is normally expressed but of which over-expression is desired. The DNA sequence may be introduced by any suitable transfection technique including electroporation, calcium phosphate precipitation, lipofection or other known to those skilled in the art. The sequence may have been introduced directly into the cell or may have been introduced into an earlier generation of the cell.

The term "transgene" means any piece of DNA which can be inserted into a cell, and preferably becomes part of the genome of the resulting organism (i.e. either stably integrated or as a stable extrachromosomal element). Such a transgene includes genes which are partly or entirely heterologous (i.e. foreign) as well as genes homologous to endogenous genes of the organism. Including within this definition is a transgene created by providing an RNA sequence with is reverse transcribed into DNA and then incorporated into the genome, or an antisense agent or molecule.

The terms "tau", "protein tau" or "tau-protein" refer to a specific (poly)peptide or protein associated with the assembly, stability, or enhancement of the polymerisation of microtubules. Tau protein exists in up to 6 different isoforms in adult brain, while only 1 isoform is expressed in fetal brain, but all are generated from a single gene on human chromosome 17 by alternative mRNA splicing . The most striking feature of tau protein, as deduced from molecular cloning, is a stretch of 31 or 32 amino acids, occurring in the carboxy-terminal part of the molecule, which can be repeated 3 or 4 times. Additional diversity is generated through 29 or 58 amino acid-long insertions in the N- terminal part of tau molecules ( Billingsley and Kincaid, 1997).
Under normal circumstances protein tau promotes microtubule assembly and stability in the axonal compartment of neurones. The microtubule-binding domain in protein tau is localised in the repeat region of tau (255-381) and is modulated by adjacent regions: the carboxyterminal tail (382-414) and the proline-rich region (143-254) . Stability and bundling of the microtubules is mediated by a short hydrophobic zipper in the carboxyterminal tail of tau Both assembly and stability are regulated by alternative mRNA splicing and phosphorylation.
In normal adult brain, protein tau contains 2 to 3 mol phosphate per mole of protein tau while phosphorylation of different sites in normal tau follows different developmental profiles (Brion, 1998). Abnormal protein tau variants of 60, 64 and 68 kDa have been detected exclusively in brain areas showing neurofibrillary changes and senile plaques (Delacourte et al, 1999). The abnormal electrophoretical behaviour of tau is due to phosphorylation since alkaline phosphatase treatment of these tau molecules changes their molecular mass to that of normal tau . Currently abnormal phosphorylation sites have been detected in PHF-tau at positions 46, 231, 235, 263 and 396 . In four of these sites, the phosphorylated residue is followed by a proline residue, indicating that a proline-directed kinase is involved in some of the abnormal phosphorylations of tau. In addition to these sites ten others are present in htau40, two of which are also abnormally phosphorylated, as indicated by antibody reactivity.
Detection of PHF-tau in brain extracts is either via antibodies or via changes in molecular weight or electrophoretic mobility. The abnormal phosphorylation of tau in Alzheimer's disease is due to a shift in the phosphatase/kinase equilibrium. In vitro several kinases can phosphorylate tau: cdc2 and cdk5-kinases, MAP kinases, glycogen synthase kinases (GSK3) among others. Phosphatases are less well studied in brain in general and hardly in Alzheimer's disease, and only phosphatase 2A has been shown to be capable in vitro to dephosphorylate the abnormally phosphorylated sites I tau.

The term " tau gene " means a tau gene, an allelic variant, minigene, a homologue thereof or a gene, that encode for htau40, for protein tau, an isoform of tau or functional homologues thereof or at least a portion thereof

As used herein, the term "minigene" refers to a heterologous gene construct wherein one or more nonessential segments of a gene are deleted with respect to the naturally-occurring gene. Typically, deleted segments are intronic sequences of at least about 100 basepairs to several kilobases, and may span up to several tens of kilobases or more. Isolation and manipulation of large (i.e., greater than about 50 kilobases) targeting constructs is frequently difficult and may reduce the efficiency of transferring the targeting construct into a host cell. Thus, it is frequently desirable to reduce the size of a targeting construct by deleting one or more nonessential portions of the gene. Typically, intronic sequences that do not encompass essential regulatory elements may be deleted. Frequently, if convenient restriction sites bound a nonessential intronic sequence of a cloned gene sequence, a deletion of the intronic sequence may be produced by: (1) digesting the cloned DNA with the appropriate restriction enzymes, (2) separating the restriction fragments (e.g., by electrophoresis), (3) isolating the restriction fragments encompassing the essential exons and regulatory elements, and (4) ligating the isolated restriction fragments to form a minigene wherein the exons are in the same linear order as is present in the germline copy of the naturally-occurring gene. Alternate methods for producing a minigene will be apparent to those of skill in the art (e.g., ligation of partial genomic clones, which encompass essential exons but which lack portions of intronic sequence). Most typically, the gene segments comprising a minigene will be arranged in the same linear order as is present in the germline gene, however, this will not always be the case. Some desired regulatory elements (e.g., enhancers, silencers) may be relatively position-insensitive, so that the regulatory element will function correctly even if positioned differently in a minigene than in the corresponding germline gene. For example, an enhancer may be located at a different distance from a promoter, in a different orientation, and/or in a different linear order. For example, an enhancer that is located 3' to a promoter in germline configuration might be located 5' to the promoter in a minigene. Similarly, some genes may have exons, which are alternatively spliced, at the RNA level, and thus a minigene may have fewer exons and/or exons in a different linear order than the corresponding germline gene and still encode a functional gene product. A cDNA encoding a gene product may also be used to construct a minigene. However, since it is often desirable that the heterologous minigene be expressed similarly to the cognate naturally-occurring non-human gene, transcription of a cDNA minigene typically is driven by a linked gene promoter and enhancer from the naturally-occurring gene. Frequently, such minigene may comprise a transcriptional regulatory sequence (e.g., promoter and/or enhancer) that confers neuron-specific or CNS-specific transcription of the minigene alpha-synuclein encoding sequences.

The term "agent" is used herein to denote a chemical compound, a mixture of chemical compounds, a biological macromolecule, or an extract made from biological materials such as bacteria, plants, fungi, or animal (particularly mammalian) cells or tissues.

As used herein, "isoform tau", "tau isoform", "isoform protein tau", " protein tau isoform" refer to a (poly)peptide or protein that is encoded by at least one exon of the tau gene

### DETAILED DESCRIPTION OF THE INVENTION

The present invention involves engineered yeast cell-lines that express protein tau, preferably human tau protein, or functional isoforms or homologues thereof, to the proteins produced by these cell-lines, to the phosphorylation pattern of tau in these cell-lines and to their applications and further it discloses a process for screening for drugs for the therapy of neurodegenerative disease or more particular of dementia of the Alzheimer type or of the frontotemporal dementia with Parkinsonism and for diagnosis of this dementia by drugs that specifically bind to such tau-proteins or that affect tau protein phosphorylation. It thus provides solution for a long felt need. Of these adult-onset dementia, Alzheimer's disease (AD) is the most common form. At present, no reliable biochemical test is available for antemortem diagnosis of AD. The disease is therefore diagnosed clinically on the basis of exclusion of other forms of dementia. The diagnosis can be confirmed neuropathologically by the demonstration of large amounts of neuritic (senile) plaques and neurofibrillary tangles (NFT) in particular brain regions, i.e. hippocampus, subiculum and enthorrhinal cortex.

Neurofibrillary tangles consist of paired helical filaments (PHF) and microtubule-associated protein tau is the major protein component of PHF and thus of NFT (Brion., 1998; Delacourte , 1999). The microtubuli-binding domain of tau is tightly associated with the core of PHF while tau peptides may represent only a small portion of the major component of PHF .

Furthermore, the novel engineered yeast cell-lines of present invention are characterised in that they specifically produce naturally occurring abnormally phosphorylated tau of which the phosphorylation state is confined to a particular specified region of the tau molecules, or recombinant non-phosphorylated tau which by treatment with proline-directed kinases can provoke the phosphorylation of, amongst others, Ser-Pro or Thr-Pro sites as specified. Proline-directed kinases such as MAP kinases , cdc2 kinases , glycogen synthase kinases and cdk5 kinases can be purified from various sources, such as genetically engineered yeast strains or can be present in brain extracts. The phosphorylation of tau by these kinases is abolished or greatly diminished when one or more of the following serines/threonines are mutated to an amino acid such as Ala: T153, T175, T181. S199, S202, T205, T212, T217, T231, S235 (Billingsley and Kincaid, 1997).

Consequently, the structure-function relation of said phosphorylated mutant tau proteins can be characterised and said yeast cell-lines will be used to define in vivo the effect on a number of specified biological processes, such as formation of mitotic bundles, of pseudo-hyphen, of scar-sites, of cell-size, of cell-growth in defined conditions, of response to external signals, agent or compound. The biological processes are also the subject of the present invention and are defined in the engineered yeast cell-lines in which the production of specified human tau proteins and of specified human protein kinases results in abnormal phosphorylation of protein tau and of its interference with micro-tubular structures and with the resulting abnormal transport and functions mediated by protein tau, as specified above, to finally result in abnormal fibrillar inclusions and aggregates, characterised by phospho-epitopes referred to as a "PHF-tau epitopes" as in the brain of patients suffering from Alzheimer's disease and other tau-opathies and dementia's (Heutink, 2000).

The expression "specified abnormally phosphorylated tau protein" corresponds to the fact that the engineered yeast cell-lines of the invention CAN produce abnormally phosphorylated tau isoforms that are defined by specified compounds such as, but not restricted to, monoclonal antibodies, without reacting or cross-reacting with normal tau proteins as present in cells, in brain or in CSF.

The expression "form a specific complex" means that the phosphorylated protein tau isoforms of the invention in the engineered yeast strains as the biological system of the invention, present as higher-molecular weight products under conditions as described or mentioned in one of the following techniques:

A very possible factor that could be involved in Alzheimer's disease is hyper-phosphorylation of tau. This can be caused by disturbed equilibrium activity of kinases and Phosphatases, which regulate the phosphorylation status of tau. Kinases involved directly in phosphorylation of tau have been identified in vitro by incubation of candidate kinases with recombinant tau produced in bacteria, and have revealed mitogen activated kinases (MAP- kinases) and glycogen synthase kinase-3β (GSK-3β), cdk5 kinase with any of his activating subunits (p70, p39, p35, p29, p25, ...) and other unidentified proline-directed kinases to be capable to phosphorylate tau on epitopes as encountered in PHF-tau (for reviews see Billingsley and Kincaid, 1997, Mandelkow and Mandelkow, 1998; Delacourte, 1999 ). These are evidently, but not the only candidate kinases that phosphorylate tau in vivo.

These kinases usually are involved in signalling pathways and require activation by some other mechanism, generally also phophorylation or de-phosphorylation, but eventually proteolytic cleavage.
The mechanisms by which GSK-3β activity is controlled is by phosphorylation on tyrosine, serine and/or threonine residues by protein kinase C (PKC). MAP kinases are activated by phosphorylation on tyrosine and threonine residues by a MAP kinase (MEK) which is self phosphorylated on serine and threonine residues by MEK kinase(s), possibly identical or related to the proto-oncogenes cRaf-1 or mos, or to the genes Stell and Byr2. These kinases can then be involved indirectly in controlling phosphorylation of tau and thereby used in the present invention to identify candidate kinases and pathways.

Modelling of Alzheimer's disease PHF-type hyperphosphorylation of tau may therefore be achieved by genetic manipulations using those kinases involved directly or indirectly in the phosphorylation of tau. The inventors observed that the deletion or introduction of GSK-3β from or into cultured yeast cells that were engineered and capable to express human tau, was particularly effective in achieving hyper-phosphorylation of tau as in brain of Alzheimer patients and in brain of double transgenic mice (tau x GSK-3β).

Accordingly, in an embodiment of the present invention, the kinase that is directly or indirectly modulating the phosphorylation of the microtubule-forming protein tau is preferably glycogen synthase kinase-3β.

Methods for screening potential therapeutic agents using cell lines are very well established and well known to those skilled in the art, as cell-based models are generally used as most early stage screening methods involving immunoassays or cell-morphological characteristics. In an embodiment of present invention example antibodies, for example, monoclonal antibodies, may be used against phosphorylated and non-phosphorylated tau epitopes for which many examples are known to those skilled in the art. For example, antibodies Tau-1 and tau-5, are mouse monoclonal antibodies that react with normal tau but not PHF-tau, while AT8, AT100, AT180, AT270, PHF1, MC1, SMI31, SMI34, SMI310, ALZ-50, among others are mouse monoclonal antibodies that recognise PHF-tau but do not react with normal tau in normal human brain.

Screening may be carried out as follows: Recombinant cells of the present invention are incubated with a potential therapeutic factor for a specified time in specified conditions, and the cells are then incubated with specified antibodies after which the extent of binding is measured as an index of the hyper-phosphorylation status of tau. Effective candidate drugs will decrease hyperphosphorylation of tau. Preferably, the screening assay is performed by ELISA since these can be performed rapidly and on a large scale using microtitre plates and (semi-)automated apparatus.

Accordingly, the present application also discloses a screening assay kit comprising:
i) engineered yeast cells
ii) an antibody, preferably a monoclonal antibody, capable of binding selectively to phosphorylated tau. Alternatively the present application also discloses a screening assay further comprising
iii) an antibody, preferably a monoclonal antibody, capable of binding selectively to unphosphorylated (normal) tau.

Advantageously the screening assay is an enzyme-linked immunosorbent assay.

The recombinant cell lines of the present invention may also serve as models for other neurodegenerative disorders in which there is evidence of abnormal cytoskeletal protein phosphorylation; these conditions include frontotemporal dementia, vascular dementia, Parkinson's disease, amyotrophic lateral sclerosis, senile dementia of the Lewy body type (SDLT), and stroke.

Accordingly, the recombinant cell lines of the present invention may be used for the study of such conditions and screening of therapeutic agents for such conditions.

The invention uses introduction and expression of tau and tau-kinases in cells that do not normally express tau, i.e. yeast cells. The cells are manipulated to express tau by the introduction of its cDNA under the control of a specified controlling promoter sequence, generally using a cloning vector, for example, a plasmid. These methods and procedures are well-known to those skilled in the art.

Human GSK-3β cDNA is preferably used in the production of constructs, as a modification of the wild type gene. For this, site-directed mutagenesis was used to substitute the serine residue at codon 9 by an alanine residue in GSK-3β, to prevent down-regulation of its activity by phosphorylation at serine 9, which is known to those in the field, to be a normal inactivation signal.

### EXAMPLES

### GENERAL METHODOLOGIES USED IN THIS INVENTION.

**Yeast deletion strains:** Genomic deletions of specific genes were made in the S. cerevisiae W303-1A strain (Thomas, B.J. and Rothstein, R.J., Cell 1989; 56: 619-630), the BY4741 strain (Brachmann et al., Yeast 1998; 14: 115-132) or the Σ1278b strain (Kron, S.J. Trends Microbiol. 1997;5:450-454) as indicated. They were obtained by PCR product-directed gene disruption as described previously (Brachmann et al., Yeast 1998; 14: 115-132) using oligonucleotides listed in Table1 and using the pRS vectors as templates for auxotrophic selectable markers. Deletions were checked by Southern Blot analysis (Sambrook et al. Molecular Cloning , a Laboratory Manual, 2nd edn. 1989; Cold Spring Harbor, N.Y.: Cold Spring Harbor Laboratory Press) or by PCR analysis. The PKA deletion strains ASY62 and ASY63 were kindly provided by S. Garrett and have been described previously (Smith A. et al., EMBO J., 1998; 17:3556-3564)

**cDNA expression constructs:** Wild-type human tau and mutant tau-P301L were transformed in yeast as recombinant constructs that contained the triose phosphate isomerase (TPI) promoter (Alber T, Kawasaki G., J Mol Appl Genet 1982;1:419-434), allowing the tau-cDNAs to be constitutively expressed. For this purpose, the tau-wt 2N/4R cDNA, the tau-P301L 2N/4R cDNA or the corresponding GFP cassettes containing the tau cDNAs fused in frame to the coding sequence of GFP, were ligated into the EcoRI-Xho1 sites of the yeast/E.coli shuttle vector pJW212 which is a derivative of pYX212 (R&D systems Europe Ltd., Abingdon, UK). The cloning of the cDNA inserts was confirmed by sequence analysis using a method based on the standard dideoxy sequence analysis (Sanger et al., Proc. Natl. Acad. Sci. USA 1977;74: 5463-5467). The resulting tau-expression plasmids were transformed into the appropriate yeast strains according to the protocol outlined by Gietz R.D. and Schiestl R.H. (Methods in Molecular and Cellular Biology 1995;5: 255-269). Transformed cells were plated on selective glucose-containing medium without uracil (SD-ura) as specified by Sherman et al. (Methods in Yeast Genetics. 1986; Cold Spring Harbor, N.Y.: Cold Spring Harbor Laboratory Press). The human GSK3β cDNA expression vector was derived from pKT10-GSK3β (Andoh et al., Mol. Cell. Biol. 2000; 20:6712-6720). This plasmid was cut with *Bsm*l*,* blunt ended with T4 DNA polymerase and ligated with a *Pvu*II-*Eco*RV fragment containing the *KanMX* gene from pUG6 (Güldener U. et al., Nucleic Acids Res. 1996; 24:2519-2524). To select the yeast transformants that contain this huGSK3β cDNA expression plasmid, cells were plated on YPD plates supplemented with kanamycin at a final concentration of 150µg per ml.

**Overexpression of PP2A**: The PP2A overexpression plasmid contains the yeast *PPH21* gene fused to the PGK promoter and the PGK terminator in pGKJW2. For this construction, the *PPH21* gene was amplified with primers that introduced a *Sma*l at the 5' end and a *Bam*HI at the 3'end of the coding sequence. The PCR product was then digested with Smal and *Bam*HI, purified and ligated into the *Sma*I and *Bgl*II restriction sites of pGKJW2. The plasmid pGKJW2 was obtained by cloning a 1.8 kb *Hind*III fragment of pMA91 that contains the PGK promoter, a *Bgl*II cloning site and the PGK terminator (Mellor et al., Gene 1983; 24:1-14) into the *Hind*III site of YEpLAC181 (Gietz R.D. and Sugino A., gene 1988;74:527-534). The original pUC19 multiple cloning site of YEpLAC181 were then removed by an *Eco*RI-*Sal*I double digest followed by T4 polymerase treatment and ligation. Subsequently, a new multiple cloning cassette containing the restriction sites for *Bam*HI, *Sma*I, *Eco*RI and *Bgl*II was introduced between the PGK promoter and the terminator by inserting a synthetic linker into the *Bg*/II site. Since pGKJW2 contains the *LEU2* gene, transformants were selected on minimal glucose-containing medium without leucine (SD-leu).

**Yeast culture:** Yeast cells were cultured in YEP medium (2% (w/v) bacto peptone, 2% (w/v) yeast extract) or in the appropriate selective medium in order to maintain plasmids in transformed strains. Media were supplemented with 4% (w/v) glucose (YPD or SD) unless otherwise specified. For pseudohyphal growth, cells were plated on nitrogen limitation medium ( 1mM asparagine, 0.17% (w/v) yeast nitrogen base without amino acids and without NH4SO4, 2% (w/v) glucose and 1.5% (w/v) agar). Cells were grown at 30°C or 25°C for different time periods as specified.

**Benomyl resistance assays:** For benomyl (Methyl 1-butylcarbamoyl-2-benzimidazolecarbamate) resistance assays, cells were grown in YPD supplemented with 5 to 60µg benomoyl as indicated. Growth was monitored either by plating serial dilutions or by measuring OD600 in a Bioscreen C Microbiology Workstation (Thermo Labsystems, Helsinki, Finland) according to the manufacturers specifications.

**Preparation of crude extracts for Western blotting**: Yeast cells were inoculated at a density of OD600 of 0.2 in 5 ml selective medium and grown for 16 hours at 30°C. One milliliter of the culture was transferred to a microcentrifuge tube and chilled on ice. The cells were rapidly harvested by centrifugation in a cooled (4°C) microcentrifuge at maximal speed for 15 s and the pellet was resuspended in 50µl prewarmed (95°C) standard SDS-PAGE sample buffer. The mixture was boiled for 15 min in order to denature and inactivate all proteinases and phosphatases and then processed by Western blot analysis as described below.

**Tau-solubility tests**: The solubility of protein tau was determined by sequential extraction of yeast cells (grown in SD-ura till an OD600 of 2) with high salt reassembly buffer (RAB:1M Sucrose, 0.1M MES pH7:0, 1mM EGTA, 0.56 mM MgSO4) supplemented with a protease inhibitor mix (COMPLETE, Roche Diagnostics GmbH), detergent containing radioimmunoprecipitation assay buffer (RIPA:50mM Tris pH8.0, 150 mNM NaCl, 5 mM EDTA, 0.5% (w/v) sodium deoxycholate, 0.1% (w/v) SDS, 1% (v/v) nonidet P40) and 70% formic acid as previously described (Ishihara T. et al., Neuron, 1999;24:751-762). Yeast cells were broken in reassembly buffer with glass beads by rigorous vortexing and the cell suspensions were centrifuged at 50.000xg. Samples of both the supernatants and the pellets were taken after each consecutive extraction and stored for quantitative Western blot analysis using Tau-5 antibodies.

**Western Blotting**: The denatured and reduced protein mixtures were separated by SDS-PAGE as performed under reducing conditions on either 4-20% linear gradient gels or on 8% or 12% homogenous gels (Novex, San Diego, CA). After electrophoresis, the proteins are electrophoretically transferred to nitro-cellulose filters (Hybond-C, Amersham, UK) or to PVDF filters (ABI, San Fransisco, CA). The filters are blocked by incubation for 1 hour in PBS with 0.05% (v/v)Tween 20 and 5% (w/v) skimmed dried milk (blocking buffer). The filters are then incubated overnight with a specified monoclonal antibody or a specified polyclonal antiserum appropriately diluted in same blocking buffer. The filters are then washed three times in Tween-PBS and treated for 1.5 h at room temperature with horseradish peroxidase-labelled rabbit anti-mouse IgG (Dakopatts, Denmark) diluted 1/3000 in blocking buffer. After three washes in Tween-PBS, streptavidine-biotinylated horseradish peroxidase complex (Amersham), diluted 1/250 in blocking buffer, is applied for 1.5 h at room temperature. Thereafter, the filters are washed three times in Tween-PBS and once in PBS. The filters are then incubated in PBS containing 0.05% (w/v) diaminobenzidine and 0.03% (v/v) hydrogen peroxide until background staining develops.
It should be clear that the formation of an immunological complex between the monoclonal antibodies and the antigen is not limited to the precise conditions described above, but that all techniques that respect the immunochemical properties of the antibody and antigen binding will produce similar formation of an immunological complex.
The phospho-epitopes are from 4 to 8 amino acids long and according to this embodiment of the invention, can also be defined chemically in solution or in solid phase according to any of the techniques well known in the art and compared to phosphorylated peptides as prepared according to techniques also known in the art.

The detection of the immunologically bound monoclonal antibody can be achieved by conventional technology known and comprised in the art, with a second antibody that itself carries a marker or a chemical or physical group as a marker.

**Monoclonal antibodies**: We used the monoclonal antibody Tau-5 (Pharmingen, San Diego, CA) that is directed against an epitope on human protein that is constitutively expressed on all human tau isoforms and independent of phosphorylation ("pan"-tau antibody). Monoclonal antibody AT-120 (Innogenetics, Ghent, Belgium) recognises a special epitope, which is situated immediately C-terminally of T-231. The reaction with protein tau is not phosphorylation-dependent in western blotting, while in immunohistochemistry on human brain AT-120 reacts only with the paired helical filaments (PHF) in the neurofibrillary tangles (NFT) in AD brain. The monoclonal antibodies AT-8 (Innogenetics, Ghent, Belgium) and AD-2 (gift from B. Pau, Lille, France) were used to detect specified epitopes on protein tau when phosphorylated. The epitopes of these monoclonal comprise Ser(P)-199 and Ser(P)-202 for AT8 and Ser(P)-396 and Ser(P)-404 for AD2. Similarly, the monoclonal antibody AT180 (Innogenetics, Ghent, Belgium) recognizes phosphorylated protein tau at Thr-181 and Thr-231.ln addition, monoclonal antibody Tau-1 (Roche Molecular Biochemicals, Mannheim, Germany) was used as its specificity is complementary to that of AT8, i.e. Tau-1 recognises the same epitope comprising Ser-199 and Ser-202 but only when these residues are not phosphorylated.

**Microscopy**: Yeast cells grown on pseudohyphae-inducing nitrogen limitation medium were scraped from plate, mixed with water and mounted on glass slides. GFP-transformed cells were grown till an OD600 of 3 in SD-ura and 3 µl culture were spotted on glass slides. Images were processed with a ZEISS-axioplan laser microscope under a 100x oil-immersion objective.

### EXAMPLE 1) EXPRESSION OF HUMAN PROTEIN TAU IN SACCHAROMYCES CEREVISIAE: Human protein tau is Phosphorylated when expressed in yeast.

To demonstrate that human protein tau, both wild-type and mutant P301L, can be expressed in yeast, we performed Western blotting on crude extracts from transformed W303-1A and BY4741wild-type strains and isogenic *mds1* strains that lack one of the yeast genes encoding a kinase homologous to the human tau-kinase GSK-3β. For detection we used the monoclonal antibodies Tau-5, which is the phosphorylation-independent "pan"-tau antibody. The cell lines W303-1A -HuTau-wt and W303-1A-*mds1*-HuTau-wt have been patent deposited (IDA deposit) at the Belgian Coordiated Collection of Microorganisms - BCCM IHEM-Collection and received the numbers of respectively, IHEM 19160 and IHEM 19161.

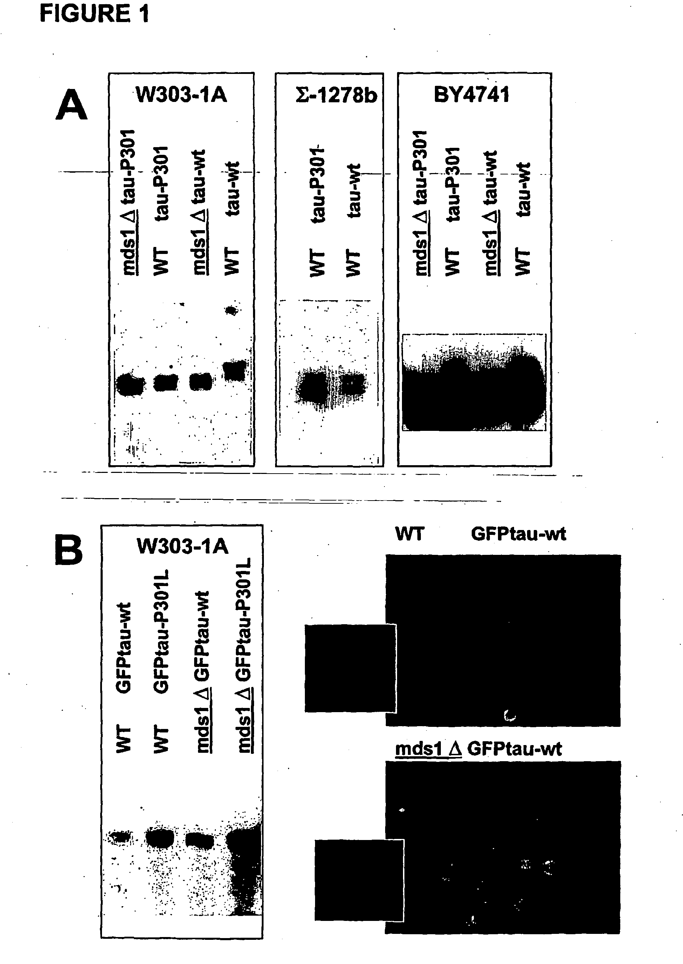

Results: As shown in Figure 1, we obtained considerable expression of human wild-type tau and human mutant tau-P301L in yeast strains, whereby its immuno-reactivity is retained as in human brain or in transgenic mouse brain, or in mammalian expression systems. This result holds true not only in the wild-type yeast strains but also in the *mds1*Δ deletion strains. In addition, the electrophoretic mobility of the immuno-reactive wild-type tau proteins is slower in the wild type strains when compared to the *mds1*Δ strains which is indicative for a difference in phosphorylation. This difference in mobility between the strains is not obvious when mutant tau-P301L is expressed suggesting that the P301 L mutation in protein tau may interfere with its phosphorylation. Furthermore, as illustrated in Figure 1B human protein tau can also be expressed as a functional GFP-fusion product without loss of immunoreactivity.

The result of present invention demonstrate the surprising finding that in a microbial eukaroytic cellular system the effect of mutations of tau on its phosphorylation can be analysed. The combination of these surprising findings constitute the needed proof-of-principle that validates the entire experimental approach and strategy to analyse protein tau, its phosphorylation and the consequences of mutations, in a less complex cellular system that is easily genetically modifiable. It is the object of present invention to provide a completely novel experimental system to address the importance of tau-mutations and the signal-transduction cascades that are responsible for and lead to hyper-phosphorylation of protein tau.

### EXAMPLE 2) EXPRESSION OF HUMAN PROTEIN TAU IN SACCHAROMYCES CEREVISIAE: Human protein tau is phosphorylated in yeast at the same phospho-epitopes as in brain of Alzheimer patients

To confirm if the heterologous expressed protein tau is post-translationally modified by phosphorylation in yeast, a more elaborated phosphorylation mapping study was performed on crude extracts obtained from the transformed W303-1A and BY4741wild type strains and their isogenic *mds1*Δ (lacking one of the yeast genes encoding a kinase homologous to the human tau-kinase GSK-3β) and *pho85*Δ strains (the latter being deficient for a kinase which is homologous to the second known tau-kinase, i.e. cdk5). As monoclonal antibodies we made use of AT-8 and AD2, which recognize only phosphorylated epitopes, and Tau-1, which detect non-phoshorylated epitopes

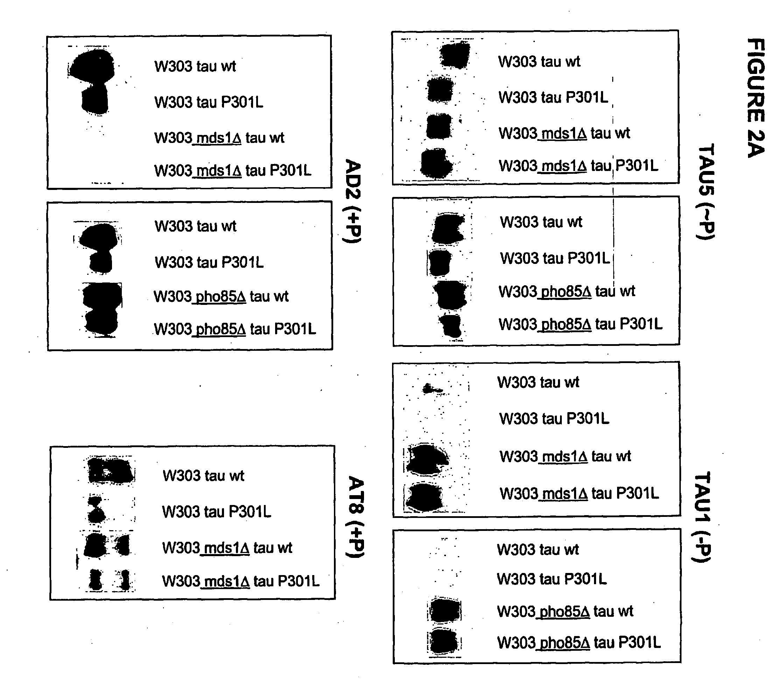

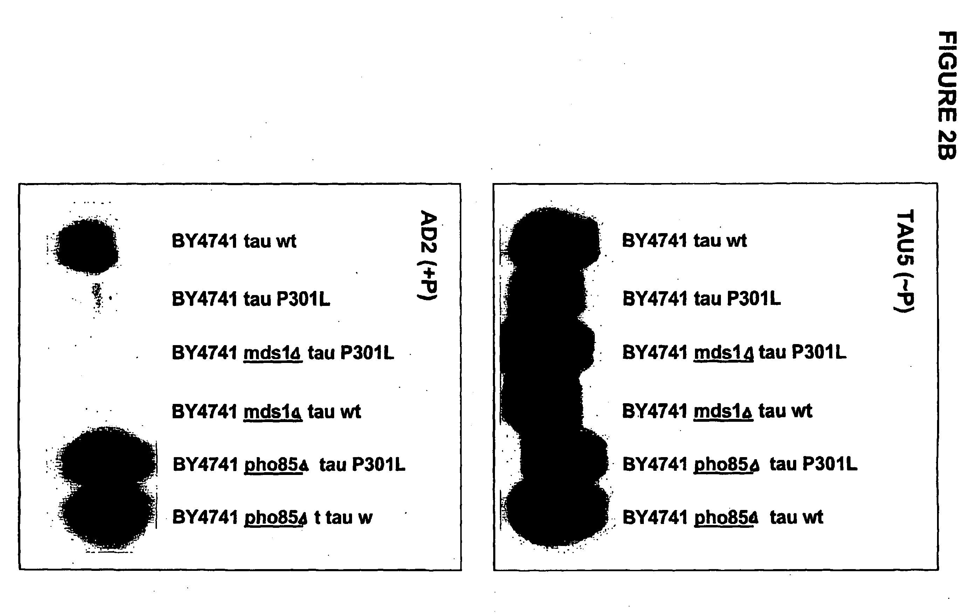

Results: The positive immunoreaction with all the different antibodies was evident in all experiments performed. Some differences in intensity in the different combinations indicated a different extent of phosphorylation as illustrated in the representative western blots shown (Figure 2).

The reactivity with AT-8 is evident when wild-type or mutant human tau is expressed, and in addition appeared independent of the genetic background of the yeast strain (Figure 2). In combination with the weak immunoreactivity of monoclonal antibody Tau-1, recognising the same epitope when not phosphorylated, these results indicate that the pathway(s) leading to the phospho-epitope defined by antibody AT8 is under-active or under-represented in the yeast strains studied here. This opens various interesting possibilities for different experimental approaches, i.e. genetic complementation, external variables, culture conditions, ... to establish these pathway(s) and define them in molecular terms.

Comparison of the immunoreactivity with antibody AT-180 in the wild type and *msd1*Δ strain demonstrates the involvement of Msd1, c.q. one of the human GSK-3β homologues in yeast, in the control of phosphorylation of Threonine-231, the phosphorylated residue that is comprised in the phospho-epitope defined by AT-180 (data not shown).

The most conspicuous result was the reaction of monoclonal antibody AD-2 that was absent in the *mds1*Δ strain but not in the *pho85*Δ strain, c.q. the human cdk5 homologue (Figure 2). This result demonstrated that the yeast Mds1 kinase is directly responsible for phosphorylation of residues Serine-396 and Serine-404 in human protein tau. This is an excellent corroboration of our findings in the brain of GSK-3β transgenic mice, in which the AD-2 epitope was implicated in the rescue of the axonopathy caused by overexpression of protein tau (Spittaels et al., J. Biol Chem 2000; 275: 41340-41349.). This interesting finding again opens various experimental possibilities for functional complementation as demonstrated below.

Combined, these results constitute the needed evidence that the heterologous expression of human protein tau in yeast results in phosphorylation at the specified phospho-epitopes that are also found in brain of Alzheimer patients. Hence, heterologous expression of human protein tau in yeast provides a system that yields or recapitulates some or all of the specified phospho-epitopes that are hypothesised to be important in the formation of neurofibrillary tangles, and thereby cause the tau-opathy and contribute to the pathology of neurodegenerative diseases like Alzheimer's disease. In addition, some of the results provide indications for completely novel experimental systems to address the signal-transduction cascade pathways that are responsible for and lead to the hyper-phosphorylation of protein tau at those specified epitopes.

### EXAMPLE 3) EXPRESSION OF HUMAN PROTEIN TAU IN SACCHAROMYCES CEREVISIAE: Heterologous expressed human protein tau is less phosphorylated in yeast strains overexpressing the protein phosphatase PP2A

To illustrate that heterologous expressed protein tau is a substrate for protein phosphatases in yeast, we monitored tau-phosphorylation in a wild-type strain and a wild-type strain with overexpression of *PPH21,* one of the yeast genes encoding the phosphatase PP2A.

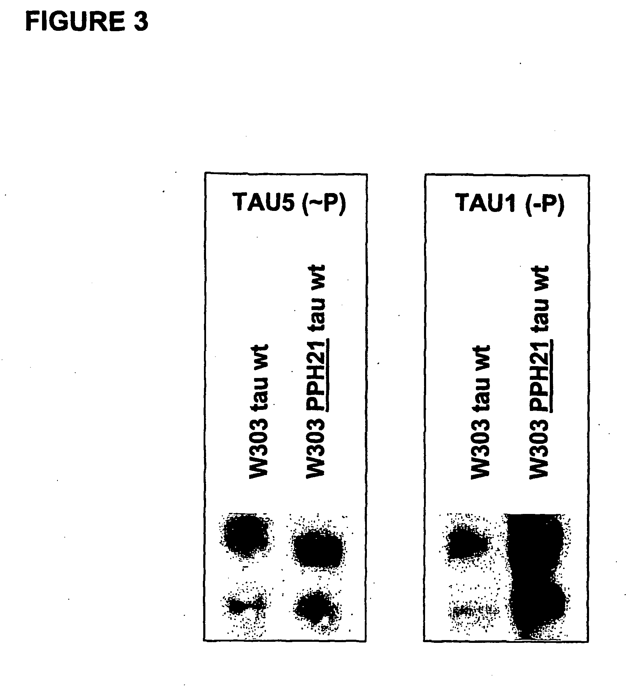

Results: As shown in Figure 3, overexpression of *PPH21* and hence increased PP2A activity, enhanced immunoreactivity of protein-tau for the antibody TAU-1 which recognizes non-phosphorylated epitopes.

In agreement to what has been suggested based on *in vitro* data with mammalian extracts, our data demonstrate that protein-tau is dephosphorylated *in vivo* when the activity of PP2A is increased in yeast. This surprising result demonstrates again that yeast can function as model study and to identify novel components involved in dephosphorylation of protein tau.

### EXAMPLE 4) EXPRESSION OF HUMAN PROTEIN TAU IN SACCHAROMYCES CEREVISIAE: Functional complementation: yeast kinases recognise the same phosphorylation sites in protein tau as their human homologues.

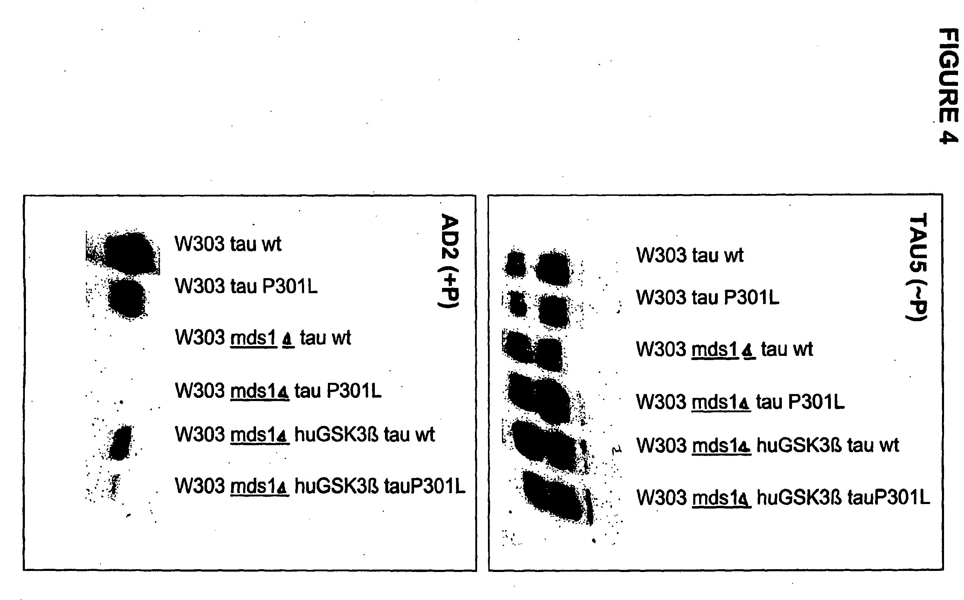

To find out if yeast kinases can be functionally complemented by and recognise the same phospho-epitopes *in vivo* as their homologous human kinases, we overexpressed the human GSK-3β in the *mds1*Δ strain (lacking one of the yeast genes encoding a kinase homologous to the human tau-kinase GSK-3β) and monitored tau-phosphorylation in crude extracts making use of monoclonal antibody AD2.
Results: As shown in Figure 4, the immunoreactivity of human protein tau to AD2 is restored in the *mds1*Δ strain when the human GSK-3β is co-expressed. This demonstrates that both the endogenous and the heterologous expressed GSK-3β kinases phosphorylate the phospho-epitopes of AD2, i.e. Ser-396 and Ser-404.

These results provide evidence for functional complementation between yeast and human signal transduction components and confirm that these evolutionary diverged proteins maintained a high degree of substrate specificity.

### EXAMPLE 5) EXPRESSION OF HUMAN PROTEIN TAU IN SACCHAROMYCES CEREVISIAE: Yeast as a model to elucidate the signal-transduction pathways that control (hyper)phosphorylation of protein tau.

To study the signal transduction cascades that control tau-phosphorylation, we monitored phosphorylation of human protein tau in strains deficient in different kinase activities, i.e PKA, Sch9 and Yak1. We and others have previously demontrated that these kinases operate in interconnecting nutrient-induced pathways in yeast (Thevelein J.M., Yeast, 1994; 10: 1753-1790; Crauwels et al., Microbiol., 1997; 143: 2627-2637; Hartley, A.D. et al., Genetics, 1994; 136: 465-474). No data are available that their human homologues, resp. PKA, PKB/Akt1 and DYRK-1, directly phosphorylate protein tau *in vivo* but several research groups reported their involvement in tau-(hyper)phosphorylation and neurodegeneration based on *in vitro* results (Bilingsley M.L. and Kincaid, R.L. Biochem. J. 1997;323: 577-591; Woods, Y.L. et al., Biochem. J. 2001; 355: 609-615).

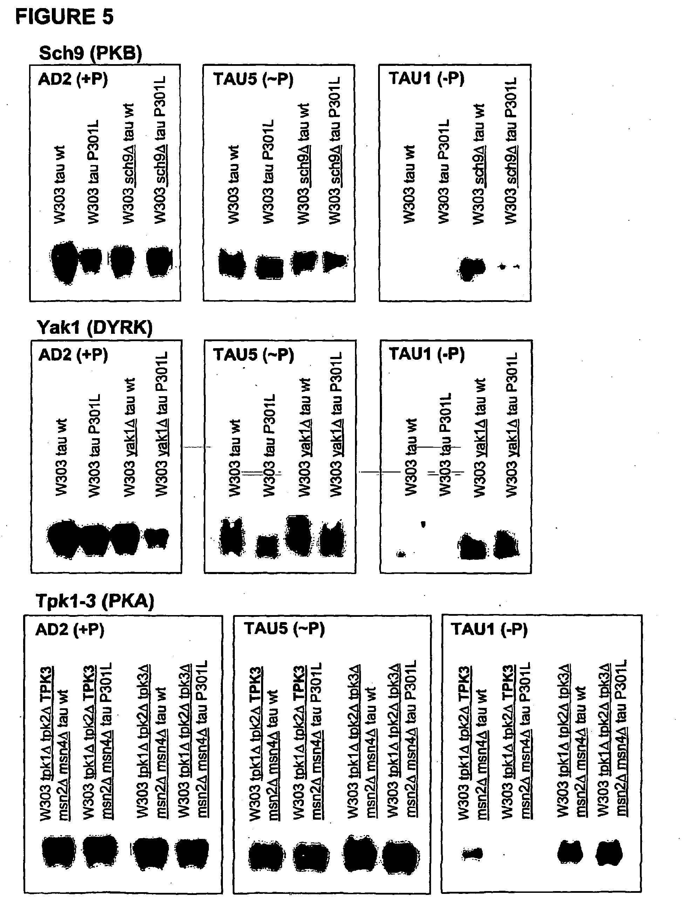

Results: The strong immunoreactivity with the monoclonal antibody Tau-1 observed upon Western blot analysis of crude extracts obtained from the different tau-expressing kinase deletion strains confirms that these kinases have a role in the phosphorylation process of tau in yeast (Figure 5).
Of particular interest is the difference in phosphorylation of wild-type tau and mutant tau- P301L in the strain without PKA activity *(tpk1*Δ *tpk2*Δ *tpk3*Δ *msn2*Δ *msn4*Δ) and the strain expressing only one of the three catalytic subunits of PKA, i.e. *TPK3 (tpk1*Δ *tpk2*Δ *TPK3 msn2*Δ *msn4*Δ). The strong immunoreactivity observed in the former, is completely abolished by the presence of Tpk3 for tau-P301 L but only partially reduced for wild-type tau. This confirms the interference of tau mutations in the phosphorylation process (cfr example 1). In addition, the limited reduction of immunoreactivity to Tau-1 of wild-type tau in the *TPK3* strain illustrates that Tpk3 can only mediate partial phosphorylation of Ser-199 and Ser-202 and that full PKA activity is required for complete phosphorylation. These findings demonstrate that engineered yeast strains can be used as a model to study the signal transduction cascade that controls tau phosphorylation.

Conclusions: These results provide the first *in vivo* data for a contribution of PKA, Sch9 (PKB/Akt) and Yak1 (DYRK-1) in the control of tau-phosphorylation. Whether this implies a direct phosphorylation, i.e. that these kinases recognise tau as a substrate, cannot be concluded but this can be addressed by further epistasis analysis. Given that all three yeast kinases can be functionally complemented by their respective mammalian homologues as we and others have reported (Geyskens, I. Et al., Nato Sci. Ser., 2000; 316: 117-126; Yan, B. et al., Yeast, 2001; 18 (S1): S273; Zhang, Z. et al., Mol. Biol. Cell., 2001; 12: 699-710), the results demonstrate that the engineered tau-expressing yeast strains can serve as *in vivo* models to unravel the signal transduction pathways and to identify novel components that control tau-phosphorylation.

### EXAMPLE 6) EXPRESSION OF HUMAN PROTEIN TAU IN SACCHAROMYCES CEREVISIAE: Heterologous expression of human protein tau affects physiological processes in yeast allowing high throughput screening procedures to be developed

To investigate how that heterologous over-expression of human protein tau in yeast affected specified physiological processes, we monitored the formation of pseudohyphae and sensitivity to the antimitotic fungicide benomyl. Under limiting growth conditions, such as growth on poor nitrogen sources, certain yeast strains undergo dimorphic transition to an apparent filamentous growth form, referred to as pseudohyphal differentiation. This not only requires changes in the budding pattern and cell adhesion but also cell elongation and hence alteration of the microfilament cytoskeleton (Gancedo, J.M., Ferns microbiol. Rev., 2001;25:107-123; Winsor B and Schiebel E, Yeast, 1997;13:399-434). Benomyl, like nocodazole, is a microtubule destabilising agent and inhibitor of microtubule polymerisation (Hoyt M.A., Cell, 1991; 66:507-517; Carminat J.L., Stearns, T., J. Cell Biololgy, 1997; 138: 629-641).

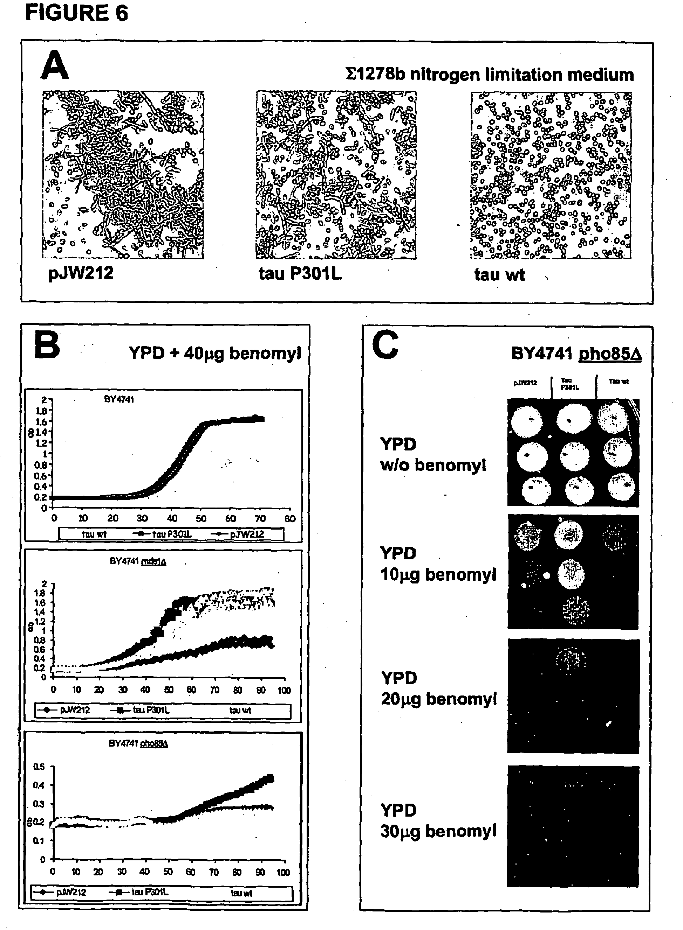

Results pseudohyphal growth: The wild-type yeast strain Σ1278b was transformed either with wild-type tau, mutant tau-P301L or an empty plasmid and grown for 4 days under conditions of nitrogen limitation to induce pseudohyphal differentiation. As seen in Figure 6A, expression of human wild-type protein tau interfered considerably or even prevented pseudohyphae formation under these conditions, while mutant tau-P301 L inhibited pseudohyphae formation to a lesser extent than wild-type tau.

Results benomyl sensitivity: When yeast BY4741strains were grown on YPD medium supplemented with benomyl, differences in sensitivity could be observed dependent on the deletion present and on whether wild-type tau or mutant tau-P301 L was expressed. As illustrated in the growth curves in Figure 6B, the wild type strain is less resistant to 40 µg/ml benomyl when wild-type tau is expressed. In the *mds1*Δ strain (lacking one of the yeast genes encoding a kinase homologous to the human tau-kinase GSK-3β), both wild-type and mutant tau-P301 L improved resistance whereas in the *pho85*Δ strain (deficient for a kinase which is homologous to the second known tau-kinase, i.e. cdk5), increased resistance was observed only with expression of mutant tau-P301 L. With the *pho85*Δ strain, this effect was also obtained when cells are plated on benomyl-containing YPD medium. Already with concentrations as low as 10 µg/ml benomyl, improved growth was observed for the *pho85*Δ strain transformed with mutant tau-P301L (Figure 6C). These results demonstrate that also in yeast the heterologous expressed tau interferes with microtubule function.

Conclusion: These results demonstrate clearly that heterologous over-expression of human protein tau affects specified measurable physiological processes in yeast. Since the differences in phenotypes depend on the expression of wild-type tau or mutant tau-P301L and on the protein kinase activities present in the strains, the data constitute evidence supporting the use of engineered yeast for high throughput screenings for compound affecting various tau-properties including tau-phosphorylation and tau-microtubule-interaction.

### EXAMPLE 7) EXPRESSION OF HUMAN PROTEIN TAU IN SACCHAROMYCES CEREVISIAE: Solubility-assay of heterologous expressed human protein tau in yeast

To analyse the solubility of heterologous expressed human protein tau, sequential extractions of yeast cells (grown in SD-ura till an OD600 of 2) with high salt reassembly buffer (RAB), detergent containing radioimmunoprecipitation assay buffer (RIPA) and 70% formic acid (FA) was performed as described. For detection, we used the monoclonal antibody Tau-5.

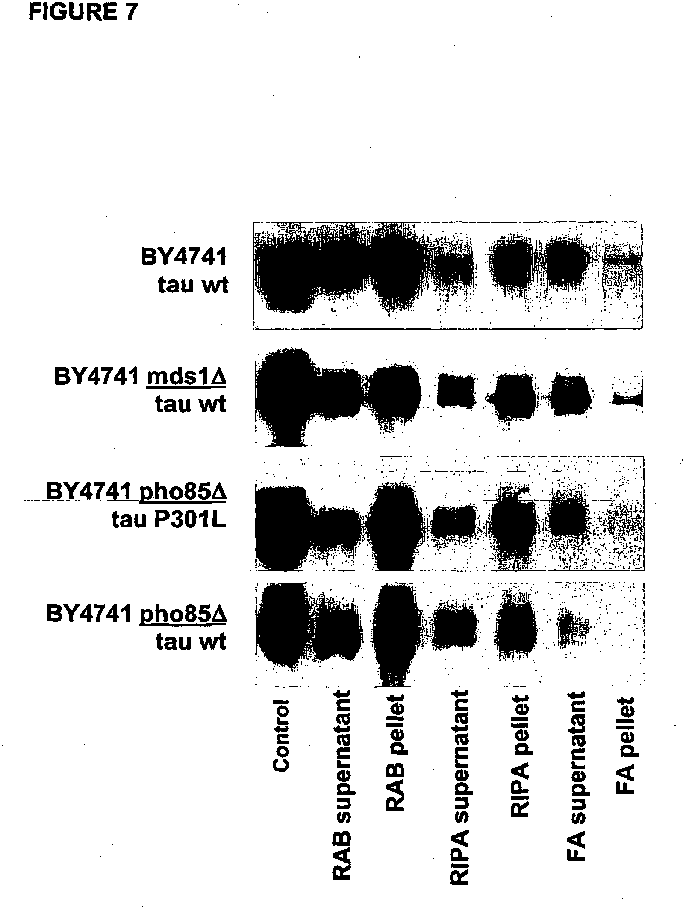

Results: As shown in Figure 7, wild type tau and mutant tau P301 L are predominantly present as insoluble protein in the yeast strains tested. Tau proteins could only be partially solubilised in RAB and RIPA buffer. With 70% formic acid, a minor fraction remained insoluble in the wild-type BY4741 and the isogenic *mds1*Δ strain but not in the *pho85*Δ mutant. In the latter, wild-type tau and mutant tau P301 L were detectable in the supernatant but not in the pellet.

It can be concluded that tau is predominantly found as insoluble protein in yeast indicating that also in this organism tau forms aggregates and that the aggregation is depending on the phosphorylation status of protein tau. Hence, yeast can be validated as model to study self assembly of tau and to elucidate the formation of paired helical filaments. In addition, such a yeast model offers the opportunity to use tau to drive the aggregation of a selectable marker and as such develop a high throughput screening strategy for components that specifically interfere with tau aggregation.

Whereas aggregation of Tau is rather time-laborious to monitor, fusion proteins of Tau with enzymatically active proteins appear to show the same phosphorylation-dependent aggregation, and in this case aggregation coincides with the removal of the corresponding enzymatic activity from the cell. This is illustrated by the fusion protein of Tau and the kanamycine-resistance gene product, which is soluble in the mds1Δ strain (lacking one of the yeast genes encoding a kinase homologous to the human tau-kinase GSK-3β), but present in an aggregated form in the wild type background. Concomitant with this observation, strains expressing the kanr-Tau fusion in a wild type background are kanamycine sensitive, whereas strains expressing kanr-Tau fusions in the mds1 background are kanamycine-resistant. Any compound inhibiting Mds1 prevents phosphorylation of the kanr-Tau fusion and hence results in kanamycine-resistance. Hence compounds which induce growth of kanr-Tau fusion expressing wild type cells on kanamycine-containing media are candidate-inhibitors of Mds1. Replacement of Mds1 with its human homologue GSK3β renders this screening assay specific for inhibitors of human GSK3β.

Compounds that cause solubilisation of the kanr-Tau fusion via a mechanism different from GSK3 inhibition will also yield a positive read-out in this assay. These GSK3-independent Tau solubilisers' are likely to represent interesting new classes of (therapeutic) compounds active against tau-o-pathies. Since monitoring of Tau expression during growth revealed that Tau expression occurred only after 5-10 hours of growth, compounds that reverse Tau aggregation as well compounds that prevent Tau aggregation can be identified depending on the time of administration of the compound to the yeast cultures. Cultures can be grown in microtiter plates and administration of compounds and monitoring of growth (optical density at 600 nm) can be fully automated, allowing the scaling up of the method and the screening of chemical libraries.

The principle of the method described can also be applied to strains expressing fusions of Tau with any other reporter, such as URA3, which has the advantage that functional Ura3 results in growth on medium without uracil, but in toxicity on FOA-containing media allowing a complementary approach and screening.

### REFERENCES TO THE APPLICATION

Billingsley ML, Kincaid RL., Biochem J 1997;323:577-591
Brion JP, Eur. Neurol., 1998, 40: 130-140.
Delacourte A Dement Geriatr Cogn Disord, 1999, 10: 75-79
DEWACHTER I., MOECHARS D., VAN DORPE J., SPITTAELS K., TESSEUR I., VAN DEN HAUTE C. and VAN LEUVEN F., Experimental Gerontology, 35, 831-841, 2000
Hardy J., Duff K, Hardy KG, Perez-Tur J, Hutton M, Nat Neurosci, 1998, 1 : 355-358
Heutink P., Hum. Molec. Genet., 2000, 9: 979-986.
Mandelkow EM and Mandelkow E, 1998, Trends Cell-Biol., 8: 425-427.
Sambrook, J. and Russel DW, 2001 "Molecular Cloning: A laboratory manual", Cold Spring Harbor Laboratory Press, Cold Spring Harbor, NY
Selkoe, D. (2000) Neurol Clin., 18: 903-922
Spittaels, K., Van den Haute, C., Van Dorpe, J., Vandezande, K., Laenen, I., Geerts, H., Mercken, M., Sciot, R., Van Lommel, A., Loos, R., Van Leuven, F., Am. J. Pathol., (1999) 155:2153-2165
Spittaels K, Van den Haute C, Van Dorpe J, Geerts H, Mercken M, Bruynseels K, Lasrado R, Vandezande K, Laenen I, Boon T, Van Lint J, Vandenheede J, Moechars D, Loos R, Van Leuven F., J. Biol Chem 2000, 275: 41340-41349.
Tesseur I., Van Dorpe J., Spittaels K., Van den Haute C., Moechars. D, Van Leuven F., Am. J. Pathol., 2000, 156, 951-964
Tesseur, I., Van Dorpe, J., Bruynseels, K., Bronfman, F., Sciot, R., Van Lommel, A. and Van Leuven, F. , Am. J. Pathol., 2000 , 157, 1495-1510.
Van Dorpe, J., Smeijers, L., Dewachter, I., Nuynens, D., Spittaels, K., Van Den Haute, C., Mercken, M., Moechars, D., Laenen, I., Kuiperi, C., Bruynseels, K., Tesseur, I., Loos, R., Vanderstichele, H., Checler, F., Sciot, R. and Van Leuven, F. Am. J. Pathol. ,2000, 157, 1283-1298.
VAN LEUVEN F., Progress in Neurobiology, 61, 305-312, 2000

**TABLE1: Oligonucleotide sequences used to delete specific genes in S. cerevisiae Sequences in bold indicate complementarity to the pRS plasmid.**

| ORF name | Oligosequence 5'-3' | |
|---|---|---|
| **MDS1** | CACGGCAACACTAATCACGCAACGC TAGAACTCGCGCAGGAGATTGTACT GAGAGTGCAC | forward |
| | TCTCCCATTATTCTTGCCTGGGCTCC CTCCGGTGCTATCACTGTGCGGTAT TTCACACCG | reversed |
| | | |
| *PHO85* | ATGTCTTCTTCTTCACAGTATGTAGT TTTCTAGTCAAGTATCATTGGAAAGT AAAGAACTAGAAATGATGAATACTA ACAAGATTGTACTGAGAGTGCAC | forward |
| | TTATGAAGCGTGGTGGTAGTACTCTG CAAACCAAGGGTGATGCAGAGCCTG CTTGGCGCTCAGCCTCATATCCGGAT TAACTGTGCGGTATTTCACACCG | reversed |
| | | |
| *SCH9* | ATGATGAATTTTTTACATCAAAATC GTCGAATCAGGATACTGGATTTAGCT CTCAACACCAACATCCAAATGGACA GAAAGATTGTACTGAGAGTGCAC | forward |
| | TCATATTTCGAATCTTCCACTGACAA ATTCGTCATCCATGTGTTGGTCGCCG TCGAAGTCCATCTGCGAATGGCTGTT GTCTGTGCGGTATTTCACACCG | reversed |
| | | |
| *YAK1* | AGTTAAGAAAAGTCAGACAAAATAA CGATTTATTCTTCGAAGATTGTACT GAGAGTGCAC | forward |
| | AGCAATCATGAACTCATCCAATAAT AACGACTCGTCCAGCCTGTGCGGTA TTTCACACCG | reversed |

## Claims

1. An engineered microbial yeast, comprising an introduced nucleotide sequence or an allelic variant, minigene, a synthetic gene or a homologue thereof coding for tau.

2. The engineered microbial yeast of claim 1, comprising an introduced mammalian nucleotide sequence or an allelic variant, minigene or a homologue thereof coding for a tau.

3. The engineered microbial yeast of claim 1 or 2, wherein tau is a wild type protein tau, a protein tau isoform, a protein tau mutant or functional homologues thereof.

4. The engineered microbial yeast of any of claims 1 to 3, wherein endogenous yeast protein kinases exhibit phosphorylation of said tau or an endogenous yeast protein phosphatase modulates phosphorylation of said tau.

5. The engineered microbial yeast of any of claims 1 to 4, that further comprises an introduced DNA sequence comprising a promoter, correctly integrated to direct the expression of a yeast kinase or phosphatase that modulates the phosphorylation of said tau.

6. The engineered microbial yeast of any of claims 1 to 5, which further comprises an introduced DNA sequence encoding a human or mammalian kinase or phosphatase that modulates the phosphorylation of said tau, under control of a promoter sequence.

7. The engineered microbial yeast of any of claims 1 to 6, which further comprises one or more of the following:
- an introduced DNA sequence comprising a promoter, correctly integrated to direct the expression of a yeast glycogen synthase kinase-3beta or a homologous yeast protein, that modulates the phosphorylation of said tau.
- an introduced DNA sequence encoding a glycogen synthase kinase-3beta or a homologous yeast protein, that modulates the phosphorylation of said tau, under control of a promoter sequence.
- an introduced DNA sequence comprising a promoter, correctly integrated to direct the expression of a yeast cdk5 or a homologous protein, that modulates the phosphorylation of said tau.
- an introduced DNA sequence encoding a cdk5 or a homologous protein, that modulates the phosphorylation of said tau under control of a promoter sequence.

8. The engineered microbial yeast of claim 7, wherein said microbial yeast when cultured exhibits phosphorylation, hyperphosphorylation or dephosphorylation of said tau.

9. The engineered microbial yeast of claim 8, which comprises at least one further kinase that can modulate the phosphorylation of tau, selected from a non-limiting group of kinases consisting of an AGC-kinase, a mitogen activated kinase, a glycogen synthase kinase, a cdk5 kinase, a cdc2 kinase, another brain proline-directed kinase, a MEK kinase, a RAS and a GEF kinase or a homologous proteins with a protein kinase function or at least one further phosphatase that can modulate the phosphorylation of tau, selected from a non-limiting group of proteins consisting of PP1 phosphatases, PP2A or PP2A-like phosphatases, PP2B phosphatases, PP2C phosphatases or other proteins with a protein phosphatase function.

10. The engineered microbial yeast of claim 1 to 9, **characterised in that** said yeast has been modulated to have modified yeast signal-transduction cascade pathways.

11. The engineered microbial yeast of claim 10, wherein said modulation results in a deletion mutant of an endogenous yeast kinase or phosphatase.

12. The engineered microbial yeast of claim 11, **characterized in that** said modulation corresponds to the deletion obtained in the MDS1Δ, the PHO85Δ, the SCH9Δ or the YAK1Δ deletion mutants.

13. The engineered microbial yeast of any of the claims 1 to 12, wherein said tau is expressed using a constitutive promoter.

14. The engineered microbial yeast of any of the claims 1 to 12, wherein tau is expressed using an inducible promoter.

15. The engineered microbial yeast of any of the claims 1 to 14, wherein the nucleotide sequence encoding tau is fused to a secretion signal.

16. The engineered microbial yeast of any of the claims 1 to 15, wherein said nucleotide sequence or allelic variant, minigene, synthetic gene or homologue thereof coding for tau is coupled in-frame to a reporter protein and is correctly integrated to allow the expression or overexpression of a reporter protein-tau fusion protein.

17. The engineered microbial yeast of claim 16, wherein tau drives the precipitation of the tau-reporter fusion protein and thereby inhibits or changes the biological function of the reporter protein.

18. The engineered microbial yeast of any of the claims 1 to 17, wherein said engineered microbial yeast is transformed with a yeast expression cDNA library whereby said cDNA is derived from human and/or mammalian tissues, including brain tissue and whereby the effect of said cDNA on tau function and/or tau phosphorylation and biochemistry is monitored.

19. The engineered microbial yeast of any one of claims 1 to 18 whereby said yeast is further modified either genetically or chemically to facilitate the uptake of agents, compounds or chemical signals.

20. The engineered microbial yeast of any of the claims 1 to 19, which is of the order of the Saccharomycetales, preferably *Saccharomyces cerevisae.*

21. The engineered microbial yeast of any one of claims 1 to 20 whereby said yeast is producing wild-type tau, tau-isoforms or tau mutants with phosphorylation status suitable for the purification and/or production thereof.

22. Use of the engineered microbial yeast of any of the claims 1 to 21 as a model for tau-opathy.

23. Use of the engineered microbial yeast of any of the claims 1 to 21 for in vivo modelling of tau biochemistry.

24. Use of the engineered microbial yeast of any of the claims 1 to 21 as a model for neurodegenerative disorders wherein aberrant phosphorylation and/or function of tau is a characteristic of said neurodegenerative disorders, preferably Alzheimer's disease or frontotemporal dementia with Parkinson's disease.

25. A method of screening a plurality of agents, compounds or chemical signals that directly or indirectly affect tau phosphorylation, function or solubility, comprising a) culturing, growing or suspending the engineered microbial yeast of any of the claims 1 to 21 in an appropriate medium, b) adding a test compound or chemical signal to said engineered microbial yeast or its medium, c) measuring the extent to which said tau phosphorylation, function and/ or solubility is affected.

26. The method of claim 25 wherein said engineered microbial yeast has been modified either genetically or chemically to facilitate the uptake of agents, compounds or chemical signals that directly or indirectly affect tau phosphorylation, function and/or solubility.

27. The method of claims 25 or 26, further comprising comparing the effect of said agents, compounds or chemical signals on said engineered microbial yeast of any of the claims 1 to 21, with the effect thereof on engineered microbial yeast that is deficient in the expression of said tau or which is deficient in the expression of a protein kinase that modulates the phosphorylation of said tau.

28. The method of any of the claims 25 to 27, further comprising comparing the effect of said agents, compounds or chemical signals on said engineered microbial yeast of any of the claims 1 to 21, with the effect thereof on engineered microbial yeast that is deficient in the expression of said tau or which is deficient in the expression of a protein phosphatase that modulates the phosphorylation of said tau.

29. The method of any of the claims 25 to 28, further comprising the use of an antibodies, preferably a monoclonal antibody, or a Fab thereof capable of binding selectively to phosphorylated or unphosphorylated tau.

30. The method according to any of the claims 25 to 29 for screening a plurality of agents, compounds or chemical signals which binds to and modulates the activity of the kinases and phosphatases that modulate the phosphorylation of said tau.

31. The method according to any of the claims 25 to 29 for screening a plurality of agents, compounds or chemical signals for binding to and modulation of the activity of any of the protein kinases of the group consisting of an AGC-kinase, a mitogen activated kinase, a glycogen synthase kinase, a cdk5 kinase, a cdc2 kinase, another brain proline-directed kinase, a MEK kinase, a RAS and a GEF kinase or a homologous protein with a protein kinase function or for screening a plurality of agents, compounds or chemical signals for binding to and modulation of the activity of any of the protein phosphatases of the group consisting of PP1 phosphatases, PP2A or PP2A-like phosphatases, PP2B phosphatases, PP2C phosphatases or other proteins with a protein phosphatase function.

32. A method of screening a plurality of agents, compounds or chemical signals for activity that directly or indirectly modulates tau phosphorylation, function or solubility comprising a) culturing, growing or suspending the engineered microbial yeast of any of the claims 1 to 21 in an appropriate medium, b) adding a test compound or chemical signal to said engineered microbial yeast or its medium, c) detecting or quantitating specified biological or cell morphogenic process in the yeast resulting from said modulation.

33. The method of claim 32, wherein said specified biological or cell morphogenic processes comprise formation of mitotic bundles, formation of pseudo-hyphen, formation of scar-sites, cell-size, cell metabolism, cell survival or cell growth in defined conditions.

34. A method for identifying an antagonist which binds and modulates the activity of an endogenous yeast kinase that modulates phosphorylation of said tau, using the screening method of any of the claims 25 to 33.

35. A method for identifying the structure-function relation of phosphorylated mutant tau proteins, wherein the method involves a) culturing, growing or suspending the engineered microbial yeast of any of the claims 1 to 21, **characterised in that** they express a mutant tau protein and a protein kinase, in an appropriate medium, b) culturing, growing or suspending the engineered microbial yeast of any of the claims 1 to 21, **characterised in that** they express said mutant tau protein but are defective in a gene coding for a protein kinase, in an appropriate medium, c) comparing specified biological processes of said kinase effective and kinase defective engineered microbial yeast.

36. A method for identifying the structure-function relation of phosphorylated mutant tau proteins, wherein the method involves a) culturing, growing or suspending the engineered microbial yeast of any of the claims 1 to 21, **characterised in that** they express a mutant tau protein and a protein phosphatase, in an appropriate medium, b) culturing, growing or suspending the engineered microbial yeast of any of the claims 1 to 21, **characterised in that** they express said mutant tau protein but are defective in a gene coding for a protein phosphatase, in an appropriate medium, c) comparing specified biological processes of said phosphatase effective and phosphatase defective engineered microbial yeast.

37. Use of claim 23 wherein said tau biochemistry relates to tau aggregation and/or tau microtubule interaction.

## Patentansprüche

1. Gentechnisch veränderte, mikrobielle Hefe, umfassend eine eingeschleuste Nukleotidsequenz oder eine allele Variante, ein Minigen, ein synthetisches Gen oder ein Homologon davon, das für Tau kodiert.

2. Gentechnisch veränderte, mikrobielle Hefe nach Anspruch 1, umfassend eine eingeschleuste Nukleotidsequenz oder eine allele Variante, ein Minigen oder ein Homologon davon, das für Tau kodiert, von einem Säuger.

3. Gentechnisch veränderte, mikrobielle Hefe nach Anspruch 1 oder 2, wobei Tau ein Wildtyp-Protein Tau, eine Isoform von Protein Tau, eine Mutante von Protein Tau oder ein funktionales Homologon davon ist.

4. Gentechnisch veränderte, mikrobielle Hefe nach einem der Ansprüche 1 bis 3, wobei endogene Hefeproteinkinasen eine Phosphorylierung von diesem Tau aufweisen oder eine endogene Hefeproteinphosphatase die Phosphorylierung von Tau moduliert.

5. Gentechnisch veränderte, mikrobielle Hefe nach einem der Ansprüche 1 bis 4, die ferner eine eingeschleuste DNA-Sequenz umfasst, die einen Promotor umfasst, der so eingebaut wurde, dass er die Expression einer Hefekinase oder -phosphatase steuert, die die Phosphorylierung von Tau moduliert.

6. Gentechnisch veränderte, mikrobielle Hefe nach einem der Ansprüche 1 bis 5, die ferner eine eingeschleuste DNA-Sequenz umfasst, die für eine humane oder Säuger-Kinase oder -Phosphatase kodiert, die die Phosphorylierung von Tau unter der Steuerung einer Promotorsequenz moduliert.

7. Gentechnisch veränderte, mikrobielle Hefe nach einem der Ansprüche 1 bis 6, die ferner eines oder mehrere der folgenden umfasst:
- eine eingeschleuste DNA-Sequenz, die einen Promotor umfasst, der so korrekt eingebaut wurde, dass er die Expression einer Hefeglycogensynthasekinase-3beta oder eines homologen Hefeproteins steuert, das die Phosphorylierung von Tau moduliert.
- eine eingeschleuste DNA-Sequenz, die für eine Glycogensynthasekinase-3beta oder ein homologes Hefeprotein kodiert, das die Phosphorylierung von Tau unter der Steuerung einer Promotorsequenz moduliert.
- eine eingeschleuste DNA-Sequenz, die einen Promotor umfasst, der so korrekt eingebaut wurde, dass er die Expression einer Hefe-cdk5 oder eines homologen Proteins steuert, das die Phosphorylierung von Tau moduliert.
- eine eingeschleuste DNA-Sequenz, die für eine cdk5 oder ein homologes Protein kodiert, das die Phosphorylierung von Tau unter der Steuerung einer Promotorsequenz moduliert.

8. Gentechnisch veränderte, mikrobielle Hefe nach Anspruch 7, wobei die mikrobielle Hefe, wenn sie kultiviert wird, eine Phosphorylierung, eine übermäßige Phosphorylierung oder eine Entphosphorylierung von Tau zeigt.

9. Gentechnisch veränderte, mikrobielle Hefe nach Anspruch 8, die wenigstens eine weitere Kinase, die die Phosphorylierung von Tau modulieren kann und ausgewählt ist aus einer nicht-einschränkenden Gruppe von Kinasen, die aus einer AGC-Kinase, einer durch Mitogen aktivierten Kinase, einer Glycogensynthasekinase, einer cdk5-Kinase, einer cdc2-Kinase einer anderen durch Prolin gesteuerten Kinase im Hirn, einer MEK-Kinase, einer RAS- und einer GEF-Kinase oder einem homologen Protein mit einer Proteinkinasefunktion besteht, oder wenigstens eine weitere Phosphatase, die die Phosphorylierung von Tau modulieren kann und ausgewählt ist aus einer nicht-einschränkenden Gruppe von Proteinen, die aus PP1-Phosphatasen, PP2A- oder PP2A-ähnlichen Phosphatasen, PP2B-Phosphatasen, PP2C-Phosphatasen oder anderen Proteinen mit einer Proteinphosphatasefunktion besteht, umfasst.

10. Gentechnisch veränderte, mikrobielle Hefe nach Anspruch 1 bis 9, **dadurch gekennzeichnet, dass** die Hefe so moduliert wurde, dass sie modifizierte Wege der Signalübertragungskaskaden von Hefe aufweist.

11. Gentechnisch veränderte, mikrobielle Hefe nach Anspruch 10, wobei die Modulation in einer Deletionsmutante einer endogenen Hefekinase oder -phosphatase resultiert.

12. Gentechnisch veränderte, mikrobielle Hefe nach Anspruch 11, **dadurch gekennzeichnet, dass** die Modulation der Deletion entspricht, die in den MDS1Δ-, den PHO85Δ-, den SCH9Δ- oder den YAK1Δ-Deletionsmutanten erhalten wurde.

13. Gentechnisch veränderte, mikrobielle Hefe nach einem der Ansprüche 1 bis 12, wobei Tau unter Verwenden eines konstitutiven Promoters exprimiert wird.

14. Gentechnisch veränderte, mikrobielle Hefe nach einem der Ansprüche 1 bis 12, wobei Tau unter Verwenden eines induzierbaren Promoters exprimiert wird.

15. Gentechnisch veränderte, mikrobielle Hefe nach einem der Ansprüche 1 bis 14, wobei die Nukleotidsequenz, die für Tau kodiert, mit einem Sekretionssignal fusioniert ist.

16. Gentechnisch veränderte, mikrobielle Hefe nach einem der Ansprüche 1 bis 15, wobei die Nukleotidsequenz oder die allele Variante, das Minigen, das synthetische Gen oder Homologon davon, das für Tau kodiert, *in frame* an ein Reporterprotein gekoppelt und so korrekt eingebaut ist, dass es die Expression oder Überexpression eines Reporterprotein-Tau-Fusionsproteins zulässt.

17. Gentechnisch veränderte, mikrobielle Hefe nach Anspruch 16, wobei Tau die Präzipitation des Tau-Reporter-Fusionsproteins steuert und **dadurch** die biologische Funktion des Reporterproteins hemmt oder verändert.

18. Gentechnisch veränderte, mikrobielle Hefe nach einem der Ansprüche 1 bis 17, wobei die gentechnisch veränderte, mikrobielle Hefe mit einer Hefeexpressions-cDNA-Genbank transformiert wurde, wobei die cDNA aus humanen und/oder Säugergeweben, einschließlich Himgewebe, stammte und wobei die Wirkung der cDNA auf die Funktion von Tau und/oder die Phosphorylierung und die Biochemie von Tau überwacht wird.

19. Gentechnisch veränderte, mikrobielle Hefe nach einem der Ansprüche 1 bis 18, wobei die Hefe entweder genetisch oder chemisch weiter modifiziert ist, um die Aufnahme von Mitteln, Verbindungen oder chemischen Signalen zu erleichtern.

20. Gentechnisch veränderte, mikrobielle Hefe nach einem der Ansprüche 1 bis 19, die von der Ordnung der Saccharomycetales, vorzugsweise *Saccharomyces cerevisiae,* ist.

21. Gentechnisch veränderte, mikrobielle Hefe nach einem der Ansprüche 1 bis 20, wobei die Hefe Wildtyp-tau, Tau-Isoformen oder Tau-Mutanten mit einem Phosphorylierungszustand produziert, der für die Reinigung und/oder Produktion derselben geeignet ist.

22. Verwendung der gentechnisch veränderten, mikrobiellen Hefe gemäß einem der Ansprüche 1 bis 21 als Modell für Tauopathie.

23. Verwendung der gentechnisch veränderten, mikrobiellen Hefe gemäß einem der Ansprüche 1 bis 21 zur in vivo-Modellbildung der Biochemie von Tau.

24. Verwendung der gentechnisch veränderten, mikrobiellen Hefe gemäß einem der Ansprüche 1 bis 21 als Modell für neurodegenerative Störungen, wobei eine anomale Phosphorylierung und/oder Funktion von Tau ein Merkmal dieser neurodegenerativen Störungen, bevorzugt Alzheimer-Krankheit oder frontotemporaler Demenz mit Parkinson-Krankheit, ist.

25. Verfahren zum Screenen nach einer Vielzahl von Mitteln, Verbindungen oder chemischen Signalen, die direkt oder indirekt die Phosphorylierung, Funktion oder Löslichkeit von Tau beeinflussen, umfassend a) das Kultivieren, Anwachsenlassen oder Suspendieren der gentechnisch veränderten, mikrobiellen Hefe gemäß einem der Ansprüche 1 bis 21 in einem geeigneten Medium, b) das Zusetzen einer Testverbindung oder eines chemischen Signals zu der gentechnisch veränderten, mikrobiellen Hefe oder zu deren Medium, c) das Messen des Ausmaßes, bis zu dem die Phosphorylierung, Funktion und/oder Löslichkeit von Tau beeinflusst wird.

26. Verfahren nach Anspruch 25, wobei die gentechnisch veränderte, mikrobielle Hefe entweder genetisch oder chemisch modifiziert wurde, um die Aufnahme von Mitteln, Verbindungen oder chemischen Signalen, die die Phosphorylierung, Funktion und/oder Löslichkeit direkt oder indirekt beeinflussen, zu erleichtern.

27. Verfahren nach Anspruch 25 oder 26, weiter umfassend das Vergleichen der Wirkung der Mittel, Verbindungen oder chemischen Signale auf die gentechnisch veränderte, mikrobielle Hefe gemäß einem der Ansprüche 1 bis 21, mit der Wirkung davon auf eine gentechnisch veränderte, mikrobielle Hefe, die eine unzureichende Expression von Tau zeigt oder eine unzureichende Expression einer Proteinkinase, die die Phosphorylierung von Tau moduliert, zeigt.

28. Verfahren nach einem der Ansprüche 25 bis 27, weiter umfassend das Vergleichen der Wirkung der Mittel, Verbindungen oder chemischen Signale auf die gentechnisch veränderte, mikrobielle Hefe gemäß einem der Ansprüche 1 bis 21, mit der Wirkung davon auf eine gentechnisch veränderte, mikrobielle Hefe, die eine unzureichende Expression von Tau zeigt oder eine unzureichende Expression einer Proteinphosphatase, die die Phosphorylierung von Tau moduliert, zeigt.

29. Verfahren nach einem der Ansprüche 25 bis 28, weiter umfassend die Verwendung eines Antikörpers, bevorzugt eines monoklonalen Antikörpers, oder eines Fab davon, der dazu in der Lage ist, selektiv an phosphoryliertes oder nicht-phosphoryliertes Tau zu binden.

30. Verfahren nach einem der Ansprüche 25 bis 29 zum Screenen nach einer Vielzahl von Mitteln, Verbindungen oder chemischen Signalen, die an Kinasen und Phosphatasen, die die Phosphorylierung von Tau modulieren, binden und die Aktivität derselben modifizieren.

31. Verfahren nach einem der Ansprüche 25 bis 29 zum Screenen nach einer Vielzahl von Mitteln, Verbindungen oder chemischen Signalen für eine Bindung an irgendeine der Proteinkinasen aus der Gruppe, bestehend aus AGC-Kinase, einer durch Mitogen aktivierten Kinase, einer Glycogensynthasekinase, einer cdk5-Kinase, einer cdc2-Kinase, einer anderen durch Prolin gesteuerten Kinase im Hirn, einer MEK-Kinase, einer RAS- und einer GEF-Kinase oder einem homologen Protein mit einer Proteinkinasefunktion, und eine Modulation der Aktivität derselben oder zum Screenen nach einer Vielzahl von Mitteln, Verbindungen oder chemischen Signalen für eine Bindung an irgendeine der Proteinphosphatasen der Gruppe, bestehend aus PP1-Phosphatasen, PP2A- oder PP2A-ähnlichen Phosphatasen, PP2B-Phosphatasen, PP2C-Phosphatasen oder anderen Proteinen mit einer Proteinphosphatasefunktion, und eine Modulation der Aktivität derselben.

32. Verfahren zum Screenen nach einer Vielzahl von Mitteln, Verbindungen oder chemischen Signalen für eine Aktivität, die die Phosphorylierung, Funktion oder Löslichkeit von Tau direkt oder indirekt moduliert, umfassend a) das Kultivieren, Anwachsenlassen oder Suspendieren der gentechnisch veränderten, mikrobiellen Hefe gemäß einem der Ansprüche 1 bis 20 in einem geeigneten Medium, b) das Zusetzen einer Testverbindung oder eines chemischen Signals zu der gentechnisch veränderten Hefe oder deren Medium, c) das Nachweisen oder Quantifizieren des bestimmten biologischen oder zellmorphogenetischen Prozesses in der Hefe, der aus der Modulation resultiert.

33. Verfahren nach Anspruch 32, wobei die bestimmten biologischen oder zellmorphogenetischen Prozesse die Bildung von mitotischen Bündeln, die Bildung von Pseudohyphen, die Bildung von Narbenstellen, die Zellgröße, den Zellstoffwechsel, das Überleben der Zelle oder das Zellwachstum unter definierten Bedingungen umfassen.

34. Verfahren zum Identifizieren eines Antagonisten, der an eine endogene Hefekinase, die die Phosphorylierung von Tau moduliert, bindet und die Aktivität derselben moduliert, unter Verwenden des Screening-Verfahrens gemäß einem der Ansprüche 25 bis 33.

35. Verfahren zum Identifizieren der Beziehung zwischen der Struktur und der Funktion phosphorylierter, mutanter Tau-Proteine, wobei das Verfahren a) das Kultivieren, Anwachsenlassen oder Suspendieren der gentechnisch veränderten Hefe gemäß einem der Ansprüche 1 bis 20, die **dadurch gekennzeichnet ist, dass** sie ein mutantes Tau-Protein und eine Proteinkinase in einem geeigneten Medium exprimiert, b) das Kultivieren, Anwachsenlassen oder Suspendieren der gentechnisch veränderten, mikrobiellen Hefe gemäß einem der Ansprüche 1 bis 20, die **dadurch gekennzeichnet ist, dass** sie ein mutantes Tau-Protein, jedoch ein fehlerhaftes Gen, das für eine Proteinkinase kodiert, in einem geeigneten Medium, exprimiert, c) das Vergleichen bestimmter biologischer Prozesse der Kinase-wirksamen und Kinase-fehlerhaften, gentechnisch veränderten, mikrobiellen Hefe einschließt.

36. Verfahren zum Identifizieren der Beziehung zwischen der Struktur und der Funktion phosphorylierter, mutanter Tau-Proteine, wobei das Verfahren a) das Kultivieren, Anwachsenlassen oder Suspendieren der gentechnisch veränderten Hefe gemäß einem der Ansprüche 1 bis 20, die **dadurch gekennzeichnet ist, dass** sie ein mutantes Tau-Protein und eine Proteinphosphatase in einem geeigneten Medium exprimiert, b) das Kultivieren, Anwachsenlassen oder Suspendieren der gentechnisch veränderten, mikrobiellen Hefe gemäß einem der Ansprüche 1 bis 20, die **dadurch gekennzeichnet ist, dass** sie ein mutantes Tau-Protein, jedoch ein fehlerhaftes Gen, das für eine Proteinphosphatase kodiert, in einem geeigneten Medium, exprimiert, c) das Vergleichen bestimmter biologischer Prozesse der Phosphatase-wirksamen und Phosphatase-fehlerhaften, gentechnisch veränderten, mikrobiellen Hefe einschließt.

37. Verwendung nach Anspruch 23, wobei die Biochemie von Tau die Aggregation von Tau und/oder die Wechselwirkung zwischen Tau und Mikrotubuli betrifft.

## Revendications

1. Levure microbienne manipulée, comprenant une séquence de nucléotides introduite ou un variant allélique, un minigène, un gène synthétique ou un homologue de celle-ci codant pour tau.

2. Levure microbienne manipulée selon la revendication 1, comprenant une séquence de nucléotides de mammifère introduite ou un variant allélique, un minigène ou un homologue de celle-ci codant pour tau.

3. Levure microbienne manipulée selon la revendication 1 ou 2, dans laquelle tau est une protéine tau de type sauvage, une isoforme de protéine tau, un mutant de protéine tau ou des homologues fonctionnels de celle -ci.

4. Levure microbienne manipulée selon l'une quelconque des revendications 1 à 3, dans laquelle des protéines kinases endogènes de levure participent à la phosphorylation de ladite tau ou une protéine phosphatase endogène de levure module la phosphorylation de ladite tau.

5. Levure microbienne manipulée selon l'une quelconque des revendications 1 à 4, qui comprend en outre une séquence d'ADN introduite comprenant un promoteur, convenablement intégré pour diriger l'expression d'une kinase ou d'une phosphatase de levure qui module la phosphorylation de ladite tau.

6. Levure microbienne manipulée selon l'une quelconque des revendications 1 à 5, qui comprend en outre une séquence d'ADN introduite codant pour une kinase ou une phosphatase humaine ou de mammifère qui module la phosphorylation de ladite tau, sous le contrôle d'une séquence promoteur.

7. Levure microbienne manipulée selon l'une quelconque des revendications 1 à 6, qui comprend en outre une ou plusieurs séquence(s) parmi celles qui suivent :
- une séquence d'ADN introduite comprenant un promoteur, convenablement intégré pour diriger l'expression d'une glycogène synthase kinase-3bêta de levure ou d'une protéine homologue de levure, qui module la phosphorylation de ladite tau.
- une séquence d'ADN introduite codant pour une glycogène synthase kinase-3bêta ou d'une protéine homologue de levure, qui module la phosphorylation de ladite tau, sous le contrôle d'une séquence promoteur.
- une séquence d'ADN introduite comprenant un promoteur, convenablement intégré pour diriger l'expression d'une cdk5 de levure ou d'une protéine homologue, qui module la phosphorylation de ladite tau.
- une séquence d'ADN introduite codant pour une cdk5 ou une protéine homologue, qui module la phosphorylation de ladite tau sous le contrôle d'une séquence promoteur.

8. Levure microbienne manipulée selon la revendication 7, ladite levure microbienne participant, lorsqu'elle est cultivée, à la phosphorylation, à l'hyperphosphorylation ou à la déphosphorylation de ladite tau.

9. Levure microbienne manipulée selon la revendication 8, qui comprend au moins une kinase supplémentaire qui peut moduler la phosphorylation de tau, choisie dans un groupe de kinases non limitatif constitué par une kinase AGC, une kinase activée par un mitogène, une glycogène synthase kinase, une kinase cdk5, une kinase cdc2, une autre kinase cérébrale dirigée contre la proline, une kinase MEK, une kinase RAS et une kinase GEF ou une protéine homologue ayant une fonction protéine kinase ou au moins une phosphatase supplémentaire qui peut moduler la phosphorylation de tau, choisie dans un groupe non limitatif de protéines constituées par des phosphatases PP1, des phosphatases PP2A ou de type PP2A, des phosphatases PP2B, des phosphatases PP2C, ou d'autres protéines ayant une fonction de protéine phosphatase.

10. Levure microbienne manipulée selon les revendications 1 à 9, **caractérisée en ce que** ladite levure a été modulée afin d'avoir des voies de la cascade de transduction de signal modifiées.

11. Levure microbienne manipulée selon la revendication 10, dans laquelle ladite modulation conduit à un mutant de délétion d'une kinase ou d'une phosphatase endogène de levure.

12. Levure microbienne manipulée selon la revendication 11, **caractérisée en ce que** ladite modulation correspond à la délétion obtenue dans les mutants de délétion MDS1Δ, PHO85Δ, SCH9Δ ou YAK1Δ.

13. Levure microbienne manipulée selon l'une quelconque des revendications 1 à 12, dans laquelle ladite tau est exprimée en utilisant un promoteur constitutif.

14. Levure microbienne manipulée selon l'une quelconque des revendications 1 à 12, dans laquelle tau est exprimée en utilisant un promoteur inductible.

15. Levure microbienne manipulée selon l'une quelconque des revendications 1 à 14, dans laquelle la séquence de nucléotides codant pour tau est fusionnée à un signal de sécrétion.

16. Levure microbienne manipulée selon l'une quelconque des revendications 1 à 15, dans laquelle ladite séquence de nucléotides ou ledit variant allélique, ledit minigène, ledit gène synthétique ou ledit homologue de celle-ci codant pour tau est couplé dans un cadre à une protéine rapporteur et est convenablement intégré afin de permettre l'expression ou la surexpression d'une protéine de fusion tau-protéine rapporteur.

17. Levure microbienne manipulée selon la revendication 16, dans laquelle tau commande la précipitation de la protéine de fusion tau-rapporteur et inhibe ou modifie ainsi la fonction biologique de la protéine rapporteur.

18. Levure microbienne manipulée selon l'une quelconque des revendications 1 à 17, ladite levure microbienne manipulée étant transformée avec une banque d'ADNc d'expression de levure, ledit ADNc étant ainsi dérivé de tissus humains et/ou de mammifère, y compris de tissu cérébral, et l'effet dudit ADNc sur la fonction de tau et/ou sur la phosphorylation et la biochimie de tau étant ainsi contrôlé.

19. Levure microbienne manipulée selon l'une quelconque des revendications 1 à 18, ladite levure étant en outre modifiée soit génétiquement, soit chimiquement pour faciliter la capture d'agents, de composés ou de signaux chimiques.

20. Levure microbienne manipulée selon l'une quelconque des revendications 1 à 19, qui appartient à l'ordre des Saccharomycetales, de préférence *Saccharomyces cerevisiae.*

21. Levure microbienne manipulée selon l'une quelconque des revendications 1 à 20, ladite levure produisant ainsi tau de type sauvage, des isoformes de tau ou des mutants de tau ayant un état de phosphorylation approprié pour la purification et/ou la production de ceux-ci.

22. Utilisation de la levure microbienne manipulée selon l'une quelconque des revendications 1 à 21 en tant que modèle de tauopathie.

23. Utilisation de la levure microbienne manipulée selon l'une quelconque des revendications 1 à 21 pour la modélisation in vivo de la biochimie de tau.

24. Utilisation de la levure microbienne manipulée selon l'une quelconque des revendications 1 à 21 en tant que modèle pour des troubles neurodégénératifs dans lesquels une phosphorylation et/ou une fonction anormale(s) de tau est une caractéristique desdits troubles neurodégénératifs, de préférence de la maladie d'Alzheimer ou d'une démence fronto-temporale avec la maladie de Parkinson.

25. Procédé de criblage d'une pluralité d'agents, de composés ou de signaux chimiques qui affectent directement ou indirectement la phosphorylation, la fonction ou la solubilité de tau, comprenant a) la culture, la croissance ou la mise en suspension de la levure microbienne manipulée selon l'une quelconque des revendications 1 à 20 dans un milieu approprié, b) l'addition d'un composé ou d'un signal chimique à tester à ladite levure microbienne manipulée ou à son milieu, c) la mesure de l'ampleur selon laquelle la phosphorylation, la fonction et/ou la solubilité de tau est/sont affectée(s).

26. Procédé selon la revendication 25, dans lequel ladite levure microbienne manipulée a été modifiée soit génétiquement, soit chimiquement pour faciliter la capture d'agents, de composés ou de signaux chimiques qui affectent directement ou indirectement la phosphorylation, la fonction et/ou la solubilité de tau.

27. Procédé selon la revendication 25 ou 26, comprenant en outre la comparaison de l'effet desdits agents, composés ou signaux chimiques sur ladite levure microbienne manipulée selon l'une quelconque des revendications 1 à 21, avec l'effet de ceux-ci sur une levure microbienne manipulée qui est déficiente dans l'expression de ladite tau ou qui est déficiente dans l'expression d'une protéine kinase qui module la phosphorylation de ladite tau.

28. Procédé selon l'une quelconque des revendications 25 ou 27, comprenant en outre la comparaison de l'effet desdits agents, composés ou signaux chimiques sur ladite levure microbienne manipulée selon l'une quelconque des revendications 1 à 21, avec l'effet de ceux-ci sur une levure microbienne manipulée qui est déficiente dans l'expression de ladite tau ou qui est déficiente dans l'expression d'une protéine phosphatase qui module la phosphorylation de ladite tau.

29. Procédé selon l'une quelconque des revendications 25 à 28, comprenant en outre l'utilisation d'un anticorps, de préférence un anticorps monoclonal, ou un Fab de celui-ci capable de se lier sélectivement à tau phosphorylée ou non phosphorylée.

30. Procédé selon l'une quelconque des revendications 25 à 29 pour cribler une pluralité d'agents, de composés ou de signaux chimiques qui se lient à et modulent l'activité des kinases et phosphatases qui modulent la phosphorylation de ladite tau.

31. Procédé selon l'une quelconque des revendications 25 à 29 pour cribler une pluralité d'agents, de composés ou de signaux chimiques en ce qui concerne la liaison à et la modulation de l'activité de l'une quelconque des protéines kinases du groupe constitué par une kinase AGC, une kinase activée par un mitogène, une glycogène synthase kinase, une kinase cdk5, une kinase cdc2, une autre kinase cérébrale dirigée contre la proline, une kinase MEK, une kinase RAS et une kinase GEF ou une protéine homologue ayant une fonction protéine kinase, ou pour cribler une pluralité d'agents, de composés ou de signaux chimiques en ce qui concerne la liaison à et la modulation de l'activité de l'une quelconque des protéines phosphatases du groupe constitué par les phosphatases PP1, les phosphatases PP2A ou de type PP2A, les phosphatases PP2B, les phosphatases PP2C, ou d'autres protéines ayant une fonction de protéine phosphatase.

32. Procédé de criblage d'une pluralité d'agents, de composés ou de signaux chimiques en ce qui concerne une activité qui module directement ou indirectement la phosphorylation, la fonction ou la solubilité de tau comprenant a) la culture, la croissance ou la mise en suspension de la levure microbienne manipulée selon l'une quelconque des revendications 1 à 21 dans un milieu approprié, b) l'addition d'un composé ou d'un signal chimique à tester à ladite levure microbienne manipulée ou à son milieu, c) la détection ou la quantification d'un processus biologique ou morphogénétique des cellules spécifié dans la levure résultant de ladite modulation.

33. Procédé selon la revendication 32, dans lequel lesdits processus biologiques ou morphogénétiques des cellules spécifiés comprennent la formation de fuseaux mitotiques, la formation de pseudohyphes, la formation de sites cicatriciels, le dimensionnement des cellules, le métabolisme cellulaire, la survie des cellules ou la croissance des cellules dans des conditions définies.

34. Procédé pour identifier un antagoniste qui se lie et module l'activité d'une kinase endogène de levure qui module la phosphorylation de ladite tau, en utilisant le procédé de criblage selon l'une quelconque des revendications 25 à 33.

35. Procédé pour identifier la relation structure-fonction de protéines tau mutantes phosphorylées, ledit procédé impliquant a) la culture, la croissance ou la mise en suspension de la levure microbienne manipulée selon l'une quelconque des revendications 1 à 21, **caractérisé en ce qu'**elles expriment une protéine tau mutante et une protéine kinase, dans un milieu approprié, b) la culture, la croissance ou la mise en suspension de la levure microbienne manipulée selon l'une quelconque des revendications 1 à 21, **caractérisé en ce qu'**elles expriment ladite protéine tau mutante mais sont défectives dans un gène codant pour une protéine kinase, dans un milieu approprié, c) la comparaison des processus biologiques spécifiés de ladite kinase efficace et de ladite levure microbienne manipulée ayant une kinase défective.

36. Procédé pour identifier la relation structure-fonction de protéines tau mutantes phosphorylées, ledit procédé impliquant a) la culture, la croissance ou la mise en suspension de la levure microbienne manipulée selon l'une quelconque des revendications 1 à 21, **caractérisé en ce qu'**elles expriment une protéine tau mutante et une protéine phosphatase, dans un milieu approprié, b) la culture, la croissance ou la mise en suspension de la levure microbienne manipulée selon l'une quelconque des revendications 1 à 21, **caractérisé en ce qu'**elles expriment ladite protéine tau mutante mais sont défectives dans un gène codant pour une protéine phosphatase, dans un milieu approprié, c) la comparaison des processus biologiques spécifiés de ladite phosphatase efficace et de ladite levure microbienne manipulée ayant une phosphatase défective.

37. Utilisation selon la revendication 23, dans laquelle ladite biochimie de tau concerne l'agrégation de tau et/ou l'interaction tau/microtubules.
